(19) Europäisches Patentamt / European Patent Office / Office européen des brevets

(11) **EP 0 917 538 B1**

(12) **FASCICULE DE BREVET EUROPEEN**

(45) Date de publication et mention de la délivrance du brevet:
**20.01.2010 Bulletin 2010/03**

(51) Int Cl.:
*C07K 14/705* *(2006.01)*   *C12N 5/10* *(2006.01)*
*C12N 15/86* *(2006.01)*   *A61K 48/00* *(2006.01)*

(21) Numéro de dépôt: **98910796.6**

(86) Numéro de dépôt international:
**PCT/FR1998/000333**

(22) Date de dépôt: **20.02.1998**

(87) Numéro de publication internationale:
**WO 1998/037098 (27.08.1998 Gazette 1998/34)**

(54) **CELLULES EUCARYOTES EXPRIMANT A LEUR SURFACE AU MOINS UNE ISOFORME D'HLA-G ET LEURS APPLICATIONS**

EUKARYOTISCHE ZELLEN DIE ZUMINDEST EIN HLA-G ISOFORM AUF IHRE OBERFLÄCHE EXPRIMIEREN UND IHRE VERWENDUNG

EUKARYOTIC CELLS EXPRESSING AT THEIR SURFACE AT LEAST AN HLA-G ISOFORM AND THEIR APPLICATIONS

(84) Etats contractants désignés:
**BE CH DE DK ES FR GB IE IT LI NL SE**

(30) Priorité: **21.02.1997 FR 9702118**

(43) Date de publication de la demande:
**26.05.1999 Bulletin 1999/21**

(73) Titulaires:
• **COMMISSARIAT A L'ENERGIE ATOMIQUE**
**75015 Paris (FR)**
• **HLA-G Technologies**
**69005 Lyon (FR)**

(72) Inventeurs:
• **CAROSELLA, Edgardo, Delfino**
**F-75016 Paris (FR)**
• **DAUSSET, Jean**
**F-75007 Paris (FR)**
• **KIRSZENBAUM, Marek**
**F-75013 Paris (FR)**
• **PAUL, Pascale**
**F-75010 Paris (FR)**
• **ROUAS-FREISS, Nathalie**
**F-75013 Paris (FR)**

(74) Mandataire: **Vialle-Presles, Marie José et al**
**Cabinet ORES**
**36, rue de St Pétersbourg**
**75008 Paris (FR)**

(56) Documents cités:
**WO-A-95/31472     WO-A-96/31604**
**WO-A-97/00085     WO-A-97/06241**
**FR-A- 2 717 498**

• **C.M. SCHMIDT ET AL.,: "Extraembryonic expression of the human MHC class I gene HLA-G in transgenic mice. Evidence for a positive regulatory region located 1 kilobase 5' to the start site of transcription" JOURNAL OF IMMUNOLOGY, vol. 151, no. 5, 1993, BETHESDA, MD, US, pages 2633-2645, XP002047493**
• **P. MOREAU ET AL.,: "Soluble HLA-G molecule. An alternatively spliced HLA-G mRNA form candidate to encode it in peripheral blood mononuclear cells and human trophoblasts" HUMAN IMMUNOLOGY, vol. 43, 1995, NEW YORK, NY, US, pages 231-236, XP002046234 cité dans la demande**
• **L. PAZMANY ET AL.,: "Protection from natural killer cell-mediated lysis by HLA-G expression on target cells" SCIENCE, vol. 274, 1996, WASHINGTON DC, US, pages 792-795, XP002046235 cité dans la demande**
• **E.D. CAROSELLA ET AL.: "HLA-G revisited" IMMUNOLOGY TODAY, vol. 17, no. 9, 1996, AMSTERDAM, NL, pages 407-409, XP004034736 cité dans la demande**
• **N.J.G. WEBSTER ET AL., : "The hormone-binding domains of the estrogen and glucocortocoid receptors contain an inducible transcription activation function" CELL, vol. 54, 15 juillet 1988, CAMBRIDGE, MASS, US, pages 199-207, XP002069634**

- N. ROUAS-FREISS ET AL.: "The alpha1 domain of HLA-G1 and HLA-G2 inhibits cytotoxicity induced by natural killer cells: Is HLA-G the public ligand for natural killer cell inhibitory receptors?" PROCEEDINGS OF THE NATIONAL ACADEMY OF SCIENCES, vol. 94, 1 mai 1997, WASHINGTON DC, US, pages 5249-5254, XP002046238

- CHUMBLEY G. ET AL: "Resistance of HLA-G and HLA-A2 Transfectants to Lysis by Decidual NK Cells" CELLULAR IMMUNOLOGY, vol. 155, 1994, pages 312-322,
- AMIOT L. ET AL: "HLA-G transcription studies during the different stages of normal and malignant hematopoiesis" TISSUE ANTIGENS, novembre 1996 (1996-11), pages 609-614,

## LISTE DE SEQUENCES

(1) INFORMATIONS GENERALES:

(i) DEPOSANT:

(A) NOM: COMMISSARIAT A L'ENERGIE ATOMIQUE
(B) RUE: 31-33 rue de la Federation
(C) VILLE: PARIS
(E) PAYS: FRANCE
(F) CODE POSTAL: 75015

(A) NOM: CAROSELLA Edgardo Delfino
(B) RUE: 23 rue George Sand
(C) VILLE: PARIS
(E) PAYS: FRANCE
(F) CODE POSTAL: 75016

(A) NOM: DAUSSET Jean
(B) RUE: 9 rue de Villersexel
(C) VILLE: PARIS
(E) PAYS: FRANCE
(F) CODE POSTAL: 75007

(A) NOM: KIRSZENBAUM Marek
(B) RUE: 32 rue du Banquier
(C) VILLE: PARIS
(E) PAYS: FRANCE
(F) CODE POSTAL: 75013

(A) NOM: PAUL Pascale
(B) RUE: 29 rue de la Grange aux Belles
(C) VILLE: PARIS
(E) PAYS: FRANCE
(F) CODE POSTAL: 75010

(A) NOM: ROUAS-FREISS Nathalie
(B) RUE: 44 Boulevard Arago
(C) VILLE: PARIS
(E) PAYS: FRANCE
(F) CODE POSTAL: 75013

(ii) TITRE DE L' INVENTION: CELLULES EUCARYOTES EXPRIMANT A LEUR SURFACE AU MOINS UNE ISOFORME D'HLA-G ET LEURS APPLICATIONS.

(iii) NOMBRE DE SEQUENCES: 2

(iv) FORME DECHIFFRABLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(v) DONNEES DE LA DEMANDE ACTUELLE: NUMERO DE LA DEMANDE: FR 97 02118

(2) INFORMATIONS POUR LA SEQ ID NO: 1:

(i) CARACTERISTIQUES DE LA SEQUENCE:

(A) LONGUEUR: 20 paires de bases
(B) TYPE: nucléotide
(C) NOMBRE DE BRINS: simple
(D) CONFIGURATION: linéaire

(ii) TYPE DE MOLECULE: Autre acide nucléique

(A) DESCRIPTION: /desc = "Amorce"

(xi) DESCRIPTION DE LA SEQUENCE: SEQ ID NO: 1:
CCCTTTGTGA CTTCAAGAAC          20

(2) INFORMATIONS POUR LA SEQ ID NO: 2:

(i) CARACTERISTIQUES DE LA SEQUENCE:

(A) LONGUEUR: 48 paires de bases
(B) TYPE: nucléotide
(C) NOMBRE DE BRINS: simple
(D) CONFIGURATION: linéaire

(ii) TYPE DE MOLECULE: Autre acide nucléique

(A) DESCRIPTION: /desc = "Amorce"

(xi) DESCRIPTION DE LA SEQUENCE: SEQ ID NO: 2:
GGATCCTAAT ACGACTCACT ATAGGGAGGT TATAGCTCAG TGGCCCAC        48

**Description**

La présente invention est relative à des cellules eucaryotes exprimant à leur surface au moins une isoforme d'HLA-G et à leurs applications, notamment pour l'obtention d'un médicament, destiné à moduler l'activité cytolytique des cellules NK dans les pathologies où ces cellules NK sont activées ou inhibées et comme modèle d'étude, notamment dans une méthode de criblage de substances anti-tumorales.

La présente invention est également relative à des animaux non-humains transgéniques exprimant de manière spécifique au moins une isoforme d'HLA-G.

Les antigènes du complexe majeur d'histocompatibilité (CMH), se divisent en plusieurs classes, les antigènes de classe I (HLA-A, HLA-B et HLA-C) qui présentent 3 domaines globulaires ($\alpha$1, $\alpha$2 et $\alpha$3), et dont le domaine $\alpha$3 est associé à la $\beta$2 microglobuline, les antigènes de classe II (HLA-DP, HLA-DQ et HLA-DR) et les antigènes de classe III (complément).

Les antigènes de classe I comprennent, outre les antigènes précités, d'autres antigènes, dits antigènes de classe I non classiques, et notamment les antigènes HLA-E, HLA-F et HLA-G ; ce dernier, en particulier, est exprimé par les trophoblastes extravilleux du placenta humain normal.

La séquence du gène HLA-G (gène HLA-6.0) a été décrite par GERAGHTY et al., (Proc. Natl. Acad. Sci. USA, 1987, 84, 9145-9149) : il comprend 4396 paires de bases et présente une organisation intron/exon homologue à celle des gènes HLA-A, -B et -C. De manière plus précise, ce gène comprend 8 exons, 7 introns et une extrémité non traduite 3' ; les 8 exons correspondent respectivement à : exon 1 : séquence signal, exon 2 : domaine extracellulaire $\alpha$1, exon 3 : domaine extracellulaire $\alpha$2, exon 4 : domaine extracellulaire $\alpha$3, exon 5 : région transmembranaire, exon 6 : domaine cytoplasmique I, exon 7 : domaine cytoplasmique II (non traduit), exon 8 : domaine cytoplasmique III (non traduit) et région 3' non traduite (GERAGHTY et al., précité; ELLIS et al., J. Immunol., 1990, 144, 731-735 ; KIRSZENBAUM M. et al., Oncogeny of hematopoiesis. Aplastic anemia Eds. E. Gluckman, L. Coulombel, Colloque INSERM/John Libbey Eurotext Ltd). Toutefois le gène HLA-G diffère des autres gènes de classe I, en ce que le codon de terminaison de traduction, en phase, est localisé au niveau du deuxième codon de l'exon 6 : en conséquence, la région cytoplasmique de la protéine codée par ce gène HLA-6.0 est considérablement plus courte que celle des régions cytoplasmiques des protéines HLA-A, -B et -C.

Ces antigènes HLA-G sont essentiellement exprimés par les cellules cytotrophoblastiques du placenta et sont considérés comme jouant un rôle dans la protection du foetus (absence de rejet par la mère). En outre, dans la mesure où l'antigène HLA-G est monomorphique, il peut également être impliqué dans la croissance ou la fonction des cellules placentaires (KOVATS et al., Science, 1990, 248, 220-223).

D'autres recherches concernant cet antigène non classique de classe I (ISHITANI et al., Proc. Natl. Acad. Sci. USA, 1992, 89, 3947-3951) ont montré que le transcrit primaire du gène HLA-G peut être épissé de plusieurs manières et produit au moins 3 ARNm matures distincts : le transcrit primaire d'HLA-G fournit une copie complète (G1) de 1 200 pb, un fragment de 900 pb (G2) et un fragment de 600 pb (G3).

Le transcrit G1 ne comprend pas l'exon 7 et correspond à la séquence décrite par ELLIS et al. (précité), c'est-à-dire qu'il code une protéine qui comprend une séquence leader, trois domaines externes, une région transmembranaire et une séquence cytoplasmique. L'ARNm G2 ne comprend pas l'exon 3, c'est-à-dire qu'il code une protéine dans laquelle les domaines $\alpha$1 et $\alpha$3 sont directement joints ; l'ARNm G3 ne contient ni l'exon 3, ni l'exon 4, c'est-à-dire qu'il code une protéine dans laquelle le domaine $\alpha$1 et la séquence transmembranaire sont directement joints.

L'épissage qui prévaut pour l'obtention de l'antigène HLA-G2 entraîne la jonction d'une adénine (A) (provenant du domaine codant $\alpha$1) avec une séquence AC (issue du domaine codant $\alpha$3), ce qui entraîne la création d'un codon AAC (asparagine) à la place du codon GAC (acide aspartique), rencontré au début de la séquence codant le domaine $\alpha$3 dans HLA-G1.

L'épissage généré pour l'obtention de HLA-G3 n'entraîne pas la formation d'un nouveau codon dans la zone d'épissage.

Les Auteurs de cet article ont également analysé les différentes protéines exprimées : les 3 ARNm sont traduits en protéine dans la lignée cellulaire .221-G.

Les Auteurs de cet article concluent à un rôle fondamental de l'HLA-G dans la protection du foetus vis-à-vis d'une réponse immune maternelle (induction d'une tolérance immune). Toutefois, il est précisé que le rôle de la protéine G3, qui ne contient pas le domaine $\alpha$3 n'est pas établi.

Certains des Inventeurs ont récemment montré l'existence d'autres formes épissées d'ARNm d'HLA-G : le transcrit HLA-G4, qui n'inclut pas l'exon 4 ; le transcrit HLA-G5, qui inclut l'intron 4, entre les exons 4 et 5, provoquant ainsi une modification du cadre de lecture, lors de la traduction de ce transcrit et en particulier l'apparition d'un codon stop, après l'acide aminé 21 de l'intron 4 ; et le transcrit HLA-G6, possédant l'intron 4, mais ayant perdu l'exon 3 (KIRSZENBAUM M. et al., Proc. Natl. Acad. Sci. USA, 1994, 91, 4209-4213 ; Demande Européenne EP 0 677 582 ; KIRSZENBAUM M. et al., Human Immunol., 1995, 43, 237-241 ; MOREAU P. et al., Human Immunol. 1995, 43, 231-236) ; ils ont également montré que ces différents transcrits sont exprimés dans plusieurs types de cellules humaines foetales et adultes, notamment dans les lymphocytes (KIRSZENBAUM M. et al., Human Immunol., 1995, précité ; MOREAU P. et al., Human Immunol. 1995, précité).

Il existe donc au moins 5 ARNm HLA-G différents qui codent potentiellement 5 isoformes d'HLA-G.

Bien que le foetus puisse être considéré comme une semiallogreffe, les cellules foetales survivent et ne sont pas rejetées, par la mère ; il est apparu que les molécules HLA-G, exprimées à la surface des trophoblastes protègent les cellules foetales de la lyse par les cellules *natural killer* (NK) maternelles (CAROSELLA E.D. et al., C.R. Acad. Sci., 318, 827-830 ; CAROSELLA E.D. et al ; Immunol. Today, 1996, 407-409).

Dans ce dernier article, CAROSELLA et al. font une revue des connaissances générales relatives à HLA-G, une protéine détectée uniquement au niveau de cellules du placenta foetal (cytotrophoblastes) et dont la <u>fonction est inconnue</u>, comme confirmé dans CHUMBLEY G. et al. (Cellular Immunology, 1994, 155, 312-322) (page 312, début du deuxième paragraphe) et dans AMIOT L. et al. (Tissue Antigens, 1996, 48, 609-614) (page 609, fin de la première colonne et début de la deuxième colonne).

CAROSELLA et al. envisagent deux fonctions immunologiques potentielles de cette protéine, qui du fait de sa localisation spécifique au niveau de cellules du placenta foetal (cytotrophoblastes), pourrait être impliquée dans la tolérance foeto-maternelle :

- une <u>fonction inhibitrice de l'activité cytotoxique des cellules NK</u>, qui permettrait de protéger les cellules HLA-G de la lyse par les cellules NK. Cette hypothèse est supportée par les résultats présentés dans CHUMBLEY G. et al. (Cellular Immunology, 1994, 155, 312-322) qui apportent la preuve formelle que des cellules humaines dépourvues de molécules HLA conventionnelles, sont protégées de la lyse par des cellules NK du placenta maternel lorsqu'elles expriment HLA-G à leur surface. Ces cellules exprimant HLA-G sont des cellules modifiées par transfection d'un clone d'ADN génomique comprenant le locus d'HLA-G.
- une fonction inhibitrice de l'activité cytotoxique des cellules T CD8, qui permettrait de protéger les cellules exprimant HLA-G de la lyse par les cellules T CD8. Cette hypothèse n'est pas confirmée par des résultats expérimentaux.

CAROSELLA et al. concluent que <u>l'activation de la régulation de l'expression</u> d'HLA-G et notamment de l'antigène soluble, pourrait constituer une nouvelle approche thérapeutique pour prévenir le rejet de greffe.

CAROSELLA et al. ne fournissent aucun moyen concret pour résoudre ce problème exposé de manière générale.

Des études antérieures ont montré que l'expression des molécules HLA-G à la surface de cellules cibles permet de protéger lesdites cellules cibles de l'activité lytique des cellules NK de la couche déciduale de l'endomètre maternel (CHUMBLEY G. et al., Cell Immunol., 1994, 155, 312-322; DENIZ G. et al., J. Immunol., 1994, 152, 4255-4261). Il est à noter que ces cellules cibles sont obtenues par transfection avec des vecteurs comprenant l'ADN génomique de HLA-G, générant potentiellement tous les transcrits alternatifs.

Les cellules NK expriment des récepteurs des molécules du CMH de classe I (*killer inhibitory receptors* ou KIR ou NKIR pour récepteurs inhibiteurs NK), qui sont responsables de l'inhibition de la cytotoxicité, lorsque ces molécules HLA, agissant comme ligands, sont reconnues par ces récepteurs ; par exemple, PAZMANY L. et al. (Science, 1996, 274, 792-795) ont montré que l'expression de HLA-G protégeait de la lyse les cellules cibles LCL 721.221 (lignée cellulaire de lymphome B), transfectées par le gène HLA-G. Ces cellules sont habituellement sensibles aux cellules NK ; ils ont en outre identifié les récepteurs sur les cellules NK qui reconnaissent HLA-G, à savoir les récepteurs NKIR1 et NKIR2, appartenant à la super-famille des immunoglobulines (p58) capables de distinguer entre deux groupes dimorphiques de molécules HLA-C ; HLA-G pourrait être le ligand naturel des récepteurs des cellules NK ; certains des Inventeurs ont, en effet, montré que les cellules NK n'expriment aucun transcrit HLA-G, ce résultat confirmant que les produits d'expression du gène HLA-G jouent vraisemblablement un rôle dans l'immuno-tolérance (TEYSSIER M. et al., Nat. Immunol., 1995, 14, 262-270).

Compte tenu du rôle important que peut jouer la molécule HLA-G aussi bien dans les pathologies où les cellules NK sont particulièrement actives (maladies autoimmunes, transplantations) ou sont au contraire inhibées (présence anormale de molécules HLA-G, notamment sur certaines tumeurs ou dans les infections virales), les Inventeurs, poursuivant leurs travaux ont trouvé, de manière surprenante, qu'il était possible d'exprimer à la surface cellulaire, une seule isoforme et de contrôler son expression quantitative et qualitative et donc d'en maîtriser son utilisation.

La présente invention a pour objet des cellules eucaryotes modifiées par au moins une copie d'un ADNc codant une isoforme de la molécule HLA-G, **caractérisée en ce qu'**elles sont modifiées par un vecteur d'expression comprenant au moins un ADNc codant une isoforme de la molécule HLA-G, sélectionnée dans le groupe constitué par HLA-G2, HLA-G3, HLA-G4, HLA-G5 et HLA-G6.

La présente invention a également pour objet des cellules eucaryotes modifiées par au moins une copie d'un ADNc codant une isoforme de la molécule HLA-G, **caractérisées en ce qu'**elles sont modifiées par un vecteur d'expression comprenant une origine de réplication appropriée, un marqueur de sélection, le promoteur viral RSV et un ADNc codant une isoforme de la molécule HLA-G, sélectionnée dans le groupe constitué par HLA-G1, HLA-G2, HLA-G3, HLA-G4, HLA-G5 et HLA-G6.

De telles cellules expriment effectivement les différentes isoformes, sous une forme glycosylée, similaire à celle rencontrée dans les conditions biologiques.

Selon un mode de réalisation avantageux desdites cellules eucaryotes transfectées, ledit ADNc code une isoforme comportant au moins un domaine extracellulaire, notamment le domaine $\alpha1$.

De manière surprenante, les cellules NK voient leur activité cytolytique inhibée, en présence desdites cellules eucaryotes ainsi transfectées, et ce quelle que soit l'isoforme exprimée (isoforme membranaire ou isoforme sécrétée).

Lesdites cellules eucaryotes peuvent être issues de n'importe quel animal et notamment être des cellules de mammifères, plus particulièrement des cellules humaines.

Aussi bien les cellules transfectées selon l'invention que les cellules eucaryotes transfectées par un vecteur exprimant l'isoforme HLA-G1 trouvent application :

- pour l'obtention d'un médicament immunomodulant, destiné à inhiber l'activité des cellules tueuses, notamment des cellules NK et/ou à inhiber la réponse allogénique primaire ; selon le type de cellules eucaryotes transfectées, on obtient un produit immunomodulant à action générale ou un produit immunomodulant à action spécifique, notamment adapté à la protection d'un organe greffé ; on peut citer à titre d'exemples non limitatifs les cellules hépatiques, les cellules rénales ou les cellules souches hématopoïétiques transfectées ; de manière inattendue, lesdites cellules eucaryotes protègent les cellules cibles de l'attaque de toutes les classes de cellules NK, et ce quelle que soit l'isoforme d'HLA-G exprimée ; elles sont particulièrement adaptées dans la prévention du rejet des greffes (allogreffes et xénogreffes), dans la prévention des avortements à répétition et dans le traitement des maladies auto-immunes, pathologies ou situations dans lesquelles les cellules tueuses sont généralement activées.
- pour l'obtention d'un médicament destiné à lever la fonction inhibitrice de la ou des isoforme(s) exprimée(s) par lesdites cellules vis-à-vis des cellules tueuses, notamment les cellules NK, dans les pathologies où ces cellules tueuses sont inhibées par la molécule du complexe majeur d'histocompatibilité de classe I HLA-G ; en effet, les tumeurs solides expriment certaines isoformes d'HLA-G ; cette expression protège ces cellules cancéreuses de la lyse induite par les cellules NK;
- pour la production de vaccins anti-tumoraux : production d'anticorps qui vont bloquer l'antigène HLA-G et réinduire ainsi l'activité des cellules NK.
- pour la production de cellules humaines aptes à être transplantées dans un organe spécifique et à protéger ce dernier de la lyse, induite par les cellules tueuses, notamment les cellules NK ;
- comme modèle d'étude de l'interaction HLA-G/cellules immunocompétentes, notamment cellules tueuses, et plus particulièrement les cellules NK, pour trouver des effecteurs susceptibles de moduler la réponse antitumorale et d'inhiber l'expression HLA-G induite dans certains cancers, tout en conservant une expression des antigènes HLA classiques ; ceci permet leur utilisation, pour l'obtention d'un médicament destiné à lever la fonction inhibitrice de la ou des isoforme(s) exprimée(s) par lesdites cellules vis-à-vis des cellules tueuses, notamment les cellules NK, dans les pathologies, telles que les cancers, où ces cellules NK sont inhibées par la molécule du complexe majeur d'histocompatibilité de classes I HLA-G ;
- pour la production de mammifères non humains transgéniques exprimant une isoforme spécifique HLA-G, aptes à produire des tissus exprimant ladite isoforme (donneurs de xénogreffe) et/ou aptes à créer des modèles d'étude de la régulation et de la fonction des différentes isoformes HLA-G, liés à la tolérance, dans les maladies autoimmunes et au cours des greffes.

Pour produire lesdits animaux non-humains transgéniques, notamment des souris transgéniques, on introduit au moins une copie d'un segment comprenant un ADNc codant une isoforme d'HLA-G, associée à un promoteur convenable, dans les cellules d'un embryon de souris à un stade précoce.

Les Inventeurs décrivent également un récepteur purifié et isolé apte à lier au moins une isoforme d'HLA-G exprimée à la surface d'une cellule cible eucaryote telle que définie ci-dessus, ledit récepteur est exprimé à la surface de lignées cellulaires T qui n'expriment aucun récepteur inhibiteur KIR1, KIR2 ou CD94, ni au moins certains récepteurs membranaires spécifiques des cellules T, la formation du complexe isoforme d'HLA-G/récépteur inhibe la lyse des cellules cibles, par lesdites lignées T.

De manière avantageuse, lesdites lignées T n'expriment pas au moins les récepteurs CD3 et $\alpha\beta$ ou n'expriment aucun récepteur membranaire spécifique des cellules T.

La lignée cellulaire leucémique T immature dénommée YT2C2 est une lignée de ce type car elle n'exprime aucun récepteur inhibiteur KIR1, KIR2 et CD94, ni les récepteurs spécifiques des cellules T, tels que les récepteurs CD3 et $\alpha\beta$.

Pour isoler ledit récepteur, il est possible :

a) soit d'incuber lesdites lignées T avec une isoforme d'HLA-G, notamment avec l'isoforme soluble HLA-G5, isoler le complexe HLA-G/récepteur formé par un traitement des lignées à la trypsine, dissocier le récepteur de la molécule HLA-G, par traitement par un agent dénaturant tel qu'un détergent, séparer le récepteur, soit en passant le mélange ainsi obtenu sur une colonne d'immunoaffinité couplée à un anticorps anti-HLA-G, et en récupérant le récepteur libre dans l'éluat, soit en soumettant ledit mélange à une électrophorèse convenable.

b) soit de cloner ledit récepteur, conformément à la méthode décrite dans Colonna M. et al., Science, 1995, 268, 405-408.

Les Inventeurs décrivent également un procédé d'étude de l'affinité de liaison d'une isoforme d'HLA-G pour un récepteur KIR ou un récepteur tel que défini ci-dessus (récepteur à activité NK), comprenant :

- la transfection d'une cellule hôte eucaryote par un vecteur d'expression comprenant un ADNc codant une isoforme d'HLA-G,
- la culture desdites cellules hôtes, de manière à ce qu'elles expriment à leur surface ladite isoforme d'HLA-G,
- la misé en contact desdites cellules eucaryotes transfectées avec des cellules tueuses, telles que les cellules NK ou des lignées T telles que définies ci-dessus, en présence de substances activant ou inhibant l'HLA-G et/ou de substances activant ou inhibant le récepteur KIR ou le récepteur à activité NK selon l'invention et
- la mesure de la quantité de complexe isoforme HLA-G/récepteur.

La présente invention a pour objet l'utilisation d'une cellule eucaryote modifiée par au moins une copie d'un ADNc codant une isoforme de la molécule HLA-G sélectionnée dans le groupe constitué par les isoformes HLA-G1, HLA-G2, HLA-G3, HLA-G4, HLA-G5 et HLA-G6, ladite cellule exprimant ladite isoforme d'HLA-G membranaire ou sécrétée, pour l'obtention d'une composition destinée à inhiber la réponse allogénique primaire, dans la prévention du rejet de greffe ; de préférence, ladite cellule exprime l'isoforme soluble HLA-G5.

Selon un mode de réalisation avantageux de ladite utilisation, ladite cellule est une cellule hépatique, une cellule rénale ou une cellule souche hématopoïétique, de préférence une cellule humaine.

Selon un autre mode de réalisation avantageux de ladite utilisation, ladite composition est destinée à être transplantée dans un tissu ou un organe à greffer.

Selon encore un autre mode de réalisation de ladite utilisation, ladite cellule est incluse dans un tissu ou un organe d'un mammifère transgénique non-humain comprenant ledit ADNc codant une isoforme de la molécule HLA-G.

La présente invention a également pour objet un produit contenant au moins une cellule eucaryote modifiée par au moins une copie d'un ADNc codant une isoforme de la molécule HLA-G sélectionnée dans le groupe constitué par les isoformes HLA-G1, HLA-G2, HLA-G3, HLA-G4, HLA-G5 et HLA-G6 et un facteur de stimulation de l'expression d'HLA-G sélectionné dans le groupe constitué par les corticoïdes et les cytokines, en tant que préparation combinée, pour une utilisation simultanée, séparée ou étalée dans le temps, dans la prévention du rejet de greffe.

Selon un mode de réalisation avantageux desdits produits, ledit facteur de stimulation est sélectionné dans le groupe constitué par l'IL-10, l'IL1-$\beta$, le TGF-$\beta$, l'hydrocortisone et la dexaméthasone.

La présente invention a également pour objet un mammifère non-humain transgénique comprenant des cellules exprimant une molécule HLA-G, **caractérisé en ce qu**'il est modifié par un vecteur d'expression comprenant au moins un ADNc codant une isoforme de la molécule HLA-G, sélectionnée dans le groupe constitué par HLA-G2, HLA-G3, HLA-G4, HLA-G5 et HLA-G6 ou un vecteur d'expression comprenant une origine de réplication appropriée, un marqueur de sélection, le promoteur viral RSV et un ADNc codant une isoforme de la molécule HLA-G, sélectionnée dans le groupe constitué par HLA-G1, HLA-G2, HLA-G3, HLA-G4, HLA-G5 et HLA-G6.

La présente invention a en outre pour objet un tissu ou organe non-humain transgénique isolé, provenant d'un mammifère comprenant au moins une copie d'un ADNc codant pour une isoforme de la molécule d'HLA-G.

La présente invention a aussi pour objet une cellule eucaryote modifiée par au moins une copie d'un ADNc codant une isoforme de la molécule HLA-G, comme médicament pour la prévention du rejet de greffe.

La présente invention a en outre pour objet une cellule d'un mammifère non-humain transgénique comprenant au moins une copie d'un ADNc codant une isoforme d'une molécule HLA-G , un vecteur d'expression comprenant au moins un ADNc codant une isoforme de la molécule HLA-G, sélectionnée dans le groupe constitué par HLA-G2, HLA-G3, HLA-G4, HLA-G5 et HLA-G6 ou un vecteur d'expression comprenant une origine de réplication appropriée, un marqueur de sélection, le promoteur viral RSV et un ADNc codant une isoforme de la molécule HLA-G, sélectionnée dans le groupe constitué par HLA-G1, HLA-G2, HLA-G3, HLA-G4, HLA-G5 et HLA-G6, pour une utilisation comme xénogreffe, dans la transplantation d'organe.

Outre les dispositions qui précèdent, l'invention comprend encore d'autres dispositions, qui ressortiront de la description qui va suivre, qui se réfère à des exemples de mise en oeuvre de la présente invention ainsi qu'aux dessins annexés dans lesquels :

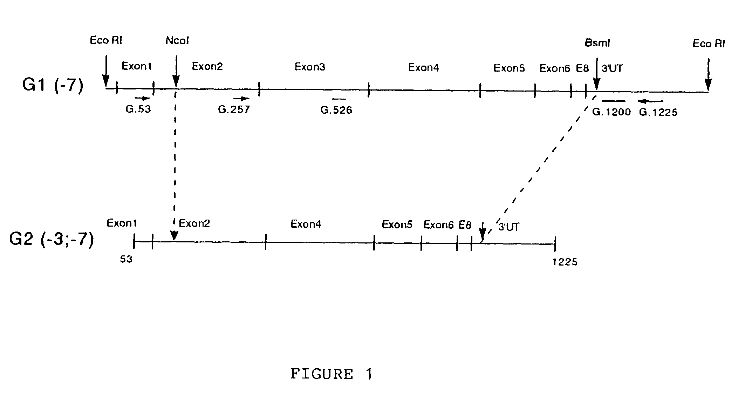

- la figure 1 illustre la construction du fragment d'ADNc HLA-G2. Les flèches verticales indiquent les sites des enzymes de restriction spécifiques pour la génération de l'ADNc HLA-G2, à partir de l'ADNc d'HLA-G1. Les flèches horizontales indiquent les amorces PCR (G.53, G.257, G.1225) et les lignes horizontales sans flèche indiquent les sondes spécifiques HLA-G utilisées pour l'hybridation (G.526 et G.1200).

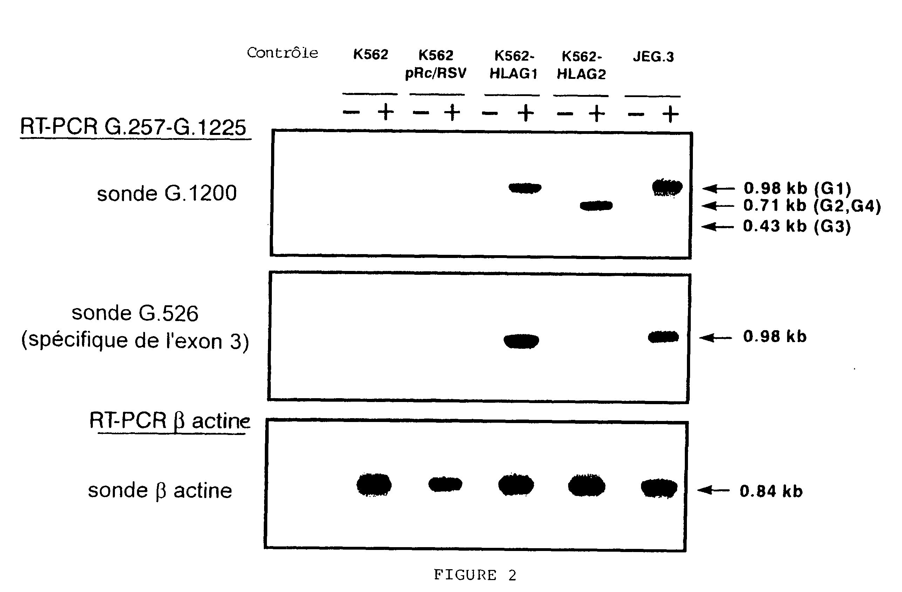

- la figure 2 illustre la détection des transcrits HLA-G dans la lignée cellulaire parentale K562, les transfectants selon l'invention et la lignée cellulaire JEG-3. Les ARN sont amplifiés en utilisant les amorces spécifiques HLA-G G.257 et G. 1225 ; le Southern blot est obtenu par hybridation avec des sondes marquées au phosphore[32] G.1200 ou G. 526. Les pistes positives (+) et négatives (-) correspondent aux matrices RT[+] et RT[-] et le blanc est un contrôle réalisé en utilisant un mélange PCR sans matrice ADNc. Pour contrôler la quantité d'ARN dans chaque échantillon, les résultats de l'amplification PCR obtenus avec des amorces spécifiques de la β-actine et un Southern blot sont hybridés avec une sonde marquée au phosphore P[32] β-actine.

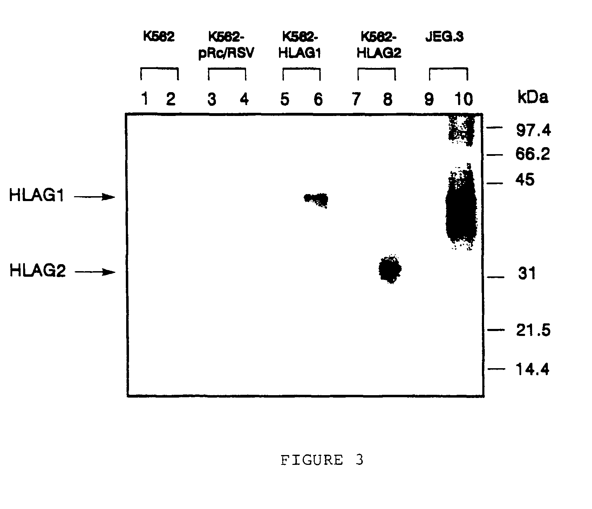

- la figure 3 illustre l'immunoprécipitation, le SDS-PAGE et l'analyse en Western blot des protéines de surface cellulaire marquées à la biotine. Des lysats membranaires biotinylés de cellules K562 (pistes 1 et 2), K562-pRc/RSV (pistes 3 et 4), K562-HLA-G1 (pistes 5 et 6), K562-HLA-G2 (pistes 7 et 8) et JEG-3 (pistes 9 et 10) sont immunoprécipités avec un anticorps monoclonal contrôle UHC-10 (pistes 1, 3, 5, 7 et 9) ou un anticorps monoclonal W6/32 (pistes 2, 4, 6, 8 et 10) et analysés par SDS-PAGE à 10% dans des conditions de réduction. Les marqueurs de poids moléculaire sont indiqués sur la droite.

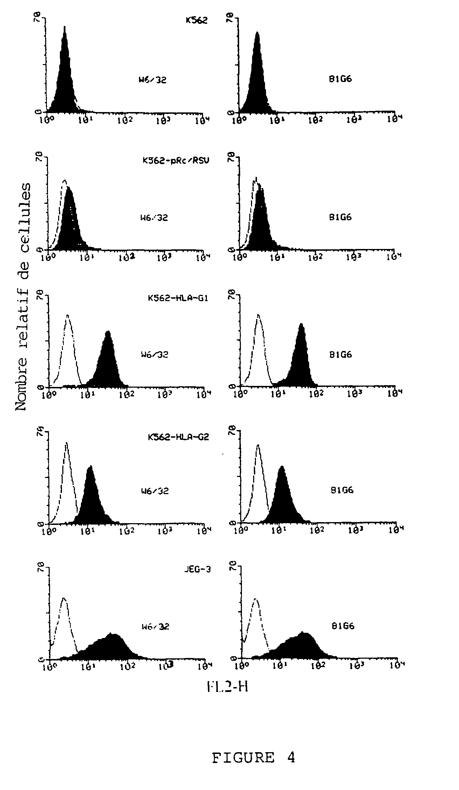

- la figure 4 illustre l'expression des molécules HLA sur les transfectants de lignée cellulaire K562 détectées par cytofluorométrie. Les cellules sauvages K562, les cellules K562 transfectées soit avec le vecteur seul (K562 pRc/RSV), soit avec les vecteurs contenant l'ADNc codant HLA-G1 (K562-HLA-G1) ou l'ADNc codant HLA-G2 (K562-HLA-G2) et les cellules JEG-3 sont marquées par immunofluorescence indirecte avec les anticorps monoclonaux primaires suivants (profils en gras) : W6/32 anticorps monomorphique anti classe I (partie gauche de la Figure) et B1.G6 anticorps anti-β2m humaine (partie droite de la Figure). Des contrôles sont réalisés sur les mêmes cellules marquées avec un anticorps contrôle (profils en blanc). Après lavage, les cellules sont marquées avec un anticorps F(ab)'2 d'immunoglobuline de chèvre anti-IgG de souris conjugué à la phycoérythrine. Les résultats ont été répétés au moins cinq fois.

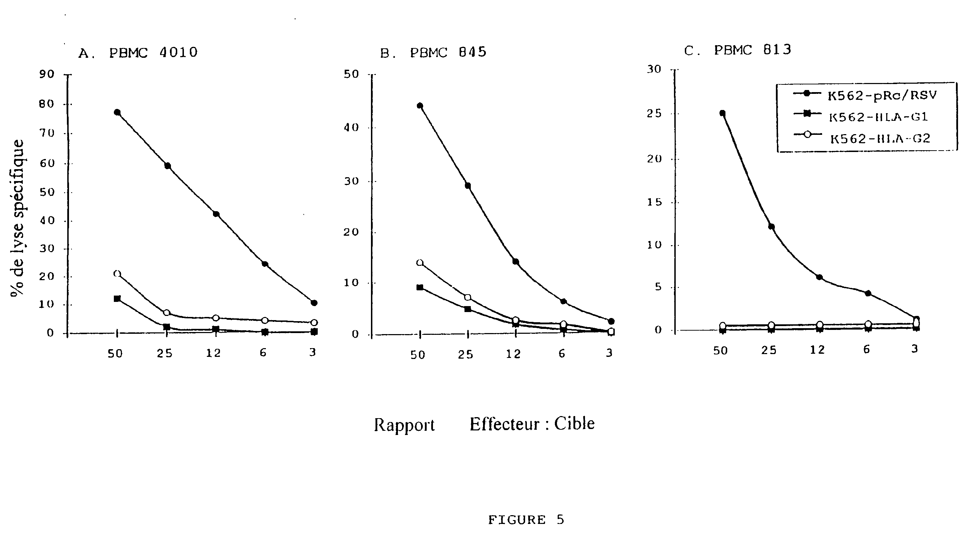

- la figure 5 illustre l'effet de l'expression de molécules HLA-G1 et HLA-G2 sur l'activité lytique des cellules NK. Des cellules K562 transfectées soit avec le vecteur seul, soit avec le vecteur HLA-G1 ou le vecteur HLA-G2 sont utilisées comme cibles (T). Des cellules mononucléées de sang périphérique (PBMC), fraîchement isolées des différents donneurs suivants : (A) donneur 4010 (HLA-A3, -B44,-B56 et -Cw1), (B) donneur 845 (HLA-A1, -A28, -B8, -B51, et -Cw7) et (C) donneur 813 (HLA-A2, -B27, -B51, et -Cw1) sont utilisées comme cellules effectrices (E). Les résultats sont exprimés en pourcentage de lyse, enregistré en 4 heures dans un test de libération du chrome 51 ($^{51}$Cr). La déviation standard de la moyenne de triplicats est en dessous de 5% et la libération spontanée n'excède jamais 10% de la libération maximale.

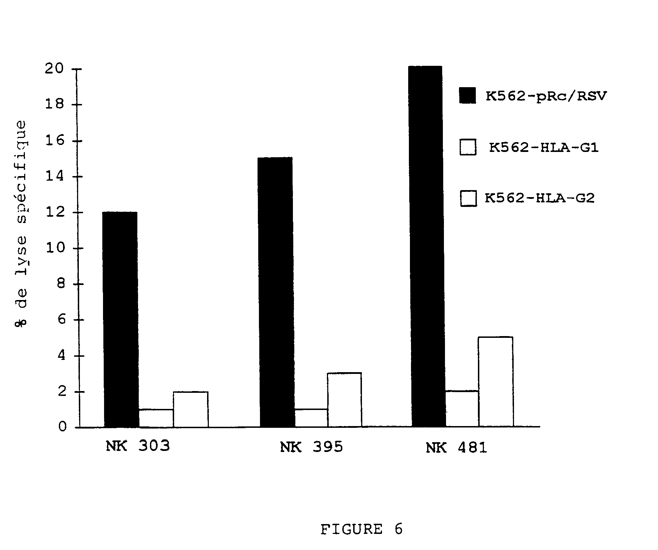

- la figure 6 illustre l'effet de l'expression des molécules HLA-G 1 et HLA-G2 sur la lyse induite par des cellules polyclonales NK. Les cellules K562 transfectées soit avec le vecteur seul, soit avec le vecteur HLA-G1, soit avec le vecteur HLA-G2 sont utilisées comme cibles (T) pour les cellules NK (dont plus de 95% ont le phénotype suivant : CD3-, CD16+ et CD56+), isolées à partir du donneur dénommé 303 (HLA-A29, -B44, -B62 et -Cw3, -Cw5) dans un rapport E:T de 1:1, du donneur dénommé 395 (HLA-A2, -B 13, -B44 et -Cw1) dans un rapport E:T de 1:1 et du donneur 481 (HLA-A3, -A26, -B27, -B35, et -Cw4, -Cw2) dans un rapport E:T de 5:1. Ces résultats sont exprimés comme pourcentage de lyse, enregistré pendant 4 heures dans un test de libération de $^{51}$Cr. La déviation standard de la moyenne de triplicats est en dessous de 5% et la libération spontanée n'excède jamais 10% de la libération maximale.

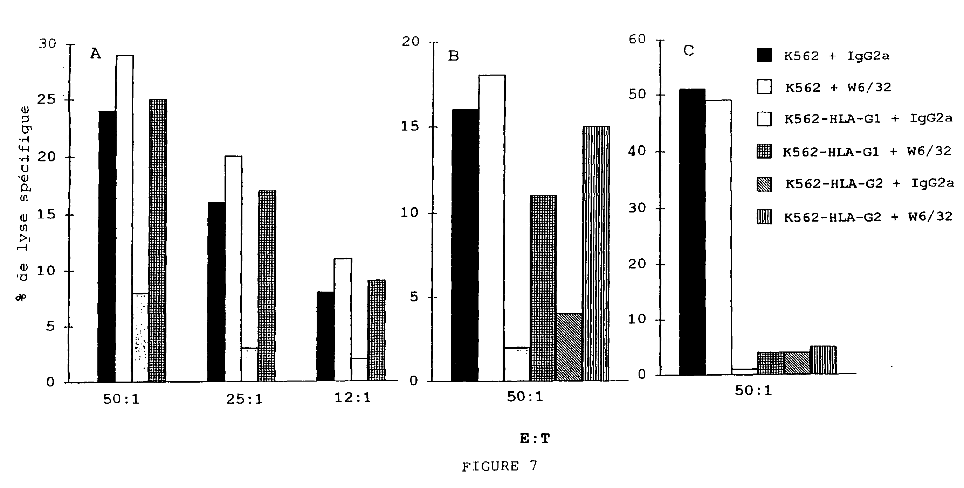

- la figure 7 illustre l'effet d'un traitement à l'aide d'un anticorps monoclonal sur la résistance des cellules cibles transfectées par un vecteur HLA-G1 ou HLA-G2 à la lyse induite par les cellules NK. La cytotoxicité est établie avec les cibles indiquées et (A) les cellules mononucléées du sang périphérique du donneur 382 (HLA-A11,-A29, -B44 et -Cw5), (B) les cellules mononucléées du sang périphérique du donneur 845 (HLA-A1, -A28, -B8, -B51, et -Cw7) et (C) le clone YT2C2, comme cellules effectrices (E). Les anticorps W6/32 ou contrôle de type IgG2a sont ajoutés à l'essai à une concentration de 10 μg/ml. Les toxicités des anticorps monoclonaux sont vérifiées pour chaque test et sont toujours inférieures à 3%.

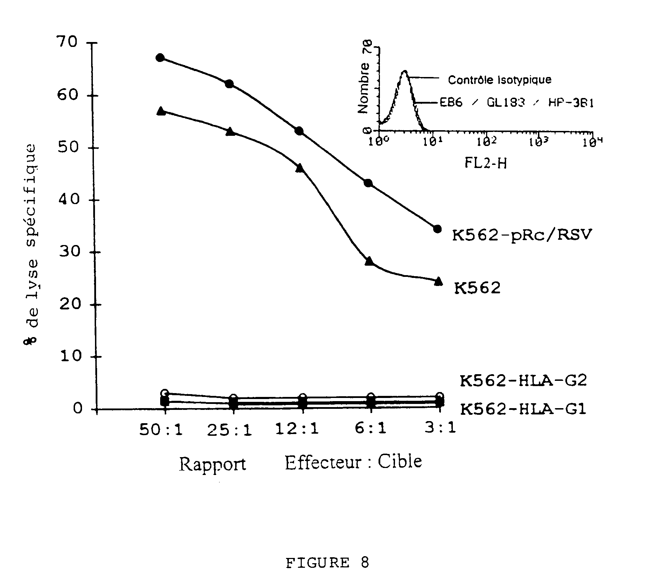

- la figure 8 illustre l'effet de l'expression HLA-G1 et HLA-G2 sur la sensibilité de la cytotoxicité du clone YT2C2. Les cellules K562 transfectées soit avec le vecteur seul, soit avec le vecteur HLA-G1 ou le vecteur HLA-G2 sont utilisées comme cellules cibles (T). Les résultats sont exprimés comme le pourcentage de lyse enregistré en 4 heures dans un test de libération du chrome 51. La déviation standard de la moyenne de triplicats est inférieure à 5% et la libération spontanée n'excède jamais 10% de la libération maximale. Cette expérience a été répétée au moins 5 fois et produit à chaque fois les mêmes résultats. L'expression des récepteurs NKIR sur le clone YT2C2 a été établie par cytofluorométrie. Les cellules YT2C2 sont marquées par immunofluorescence indirecte avec les anticorps monoclonaux primaires suivants : EB6 (IgG1, anti-p58.1 ou NKIR1), GL183 (IgG1, anti-p58.2 ou NKIR2) et HP-3B1 (IgG2a, anti-CD94). Les contrôles sont les mêmes cellules marquées avec un anticorps non pertinent. Après lavage, les cellules sont marquées à l'aide d'un conjugué F(ab)'2 d'anticorps de chèvre anti-IgG de souris/phycoérythrine. Seulement un des quatre essais est illustré sur cette figure.

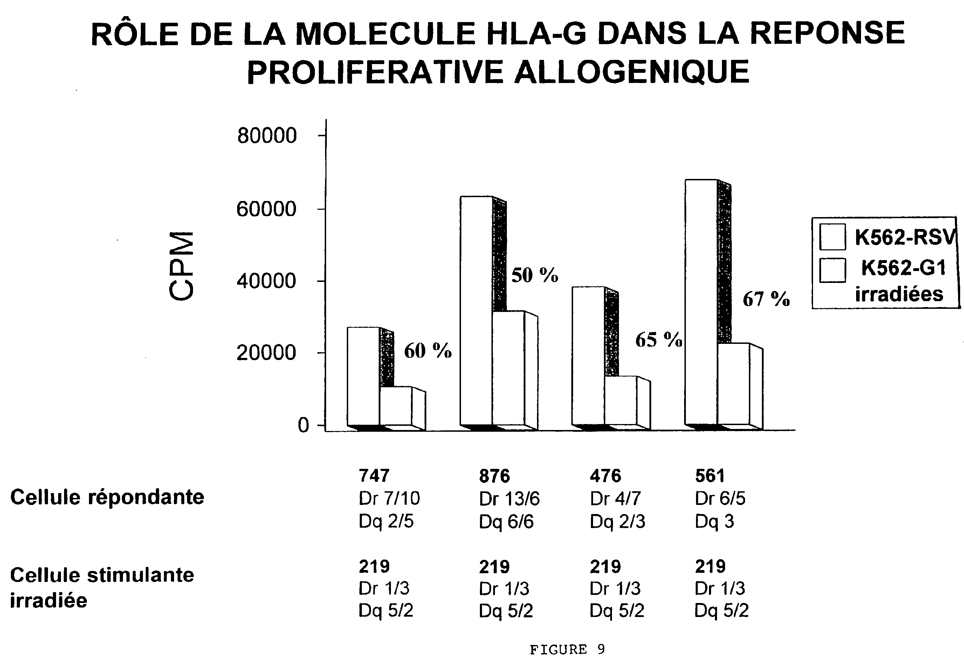

- la figure 9 illustre l'activité inhibitrice des isoformes d'HLA-G dans la réponse proliférative allogénique.

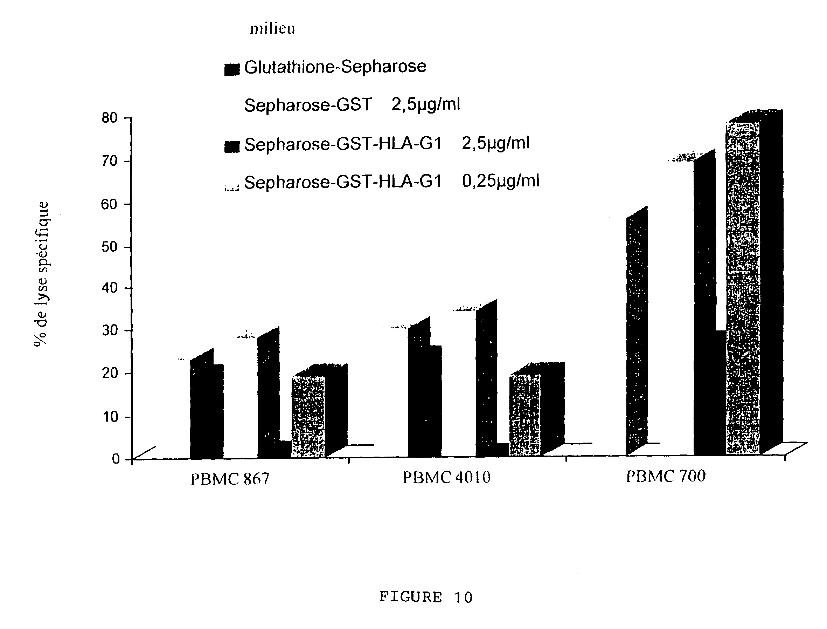

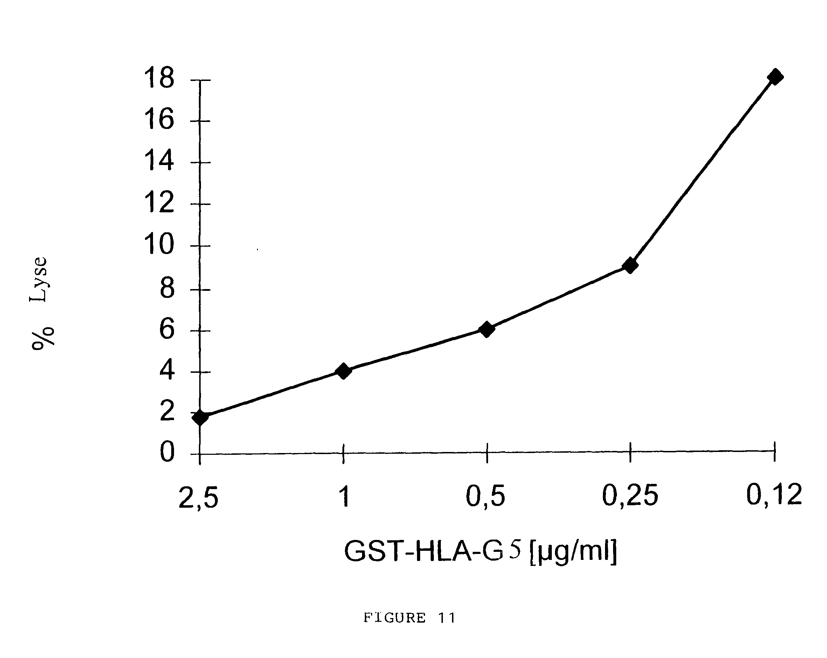

- les figures 10 et 11 illustrent l'inhibition de l'activité NK par l'isoforme HLA-G5, sous la forme d'une protéine de fusion avec la glutathion S-transférase (GST-HLA-G5). A la figure 10, des billes de Sépharose-GST-HLA-G5 sont utilisées à une concentration initiale estimée à 25 mg/ml dans un tampon PBS 1X. Les milieux de contrôle sont supplémentés avec du PBS 1X, pour remplacer l'échantillon. Les cellules mononucléées du sang périphérique du donneur 867 (HLA-A2, -A30, -B 13, -B27 et Cw6), du donneur 4010 (HLA-A3,-B44, -B56 et Cw1) et du donneur 700 (HLA-A1, -A30, -B8, -B 13 et Cw7) sont utilisés comme cellules effectrices (E). Les cellules K562 sont utilisées comme cellules cibles (T). Le rapport E:T=50. Les résultats sont exprimés comme précisé ci-dessus, en pourcentage de lyse, enregistré en 4 heures dans un test de libération du chrome 51 ($^{51}$Cr).

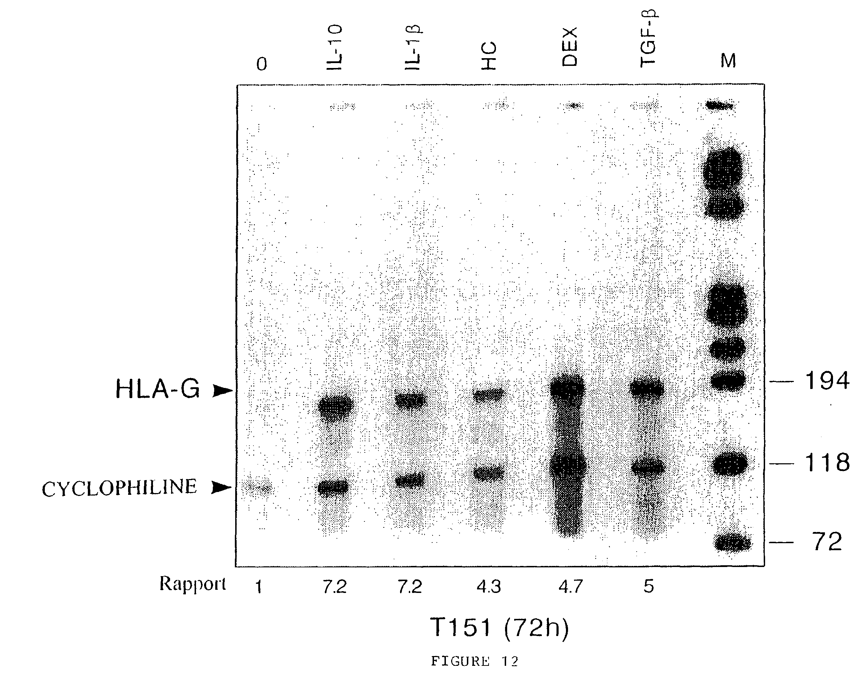

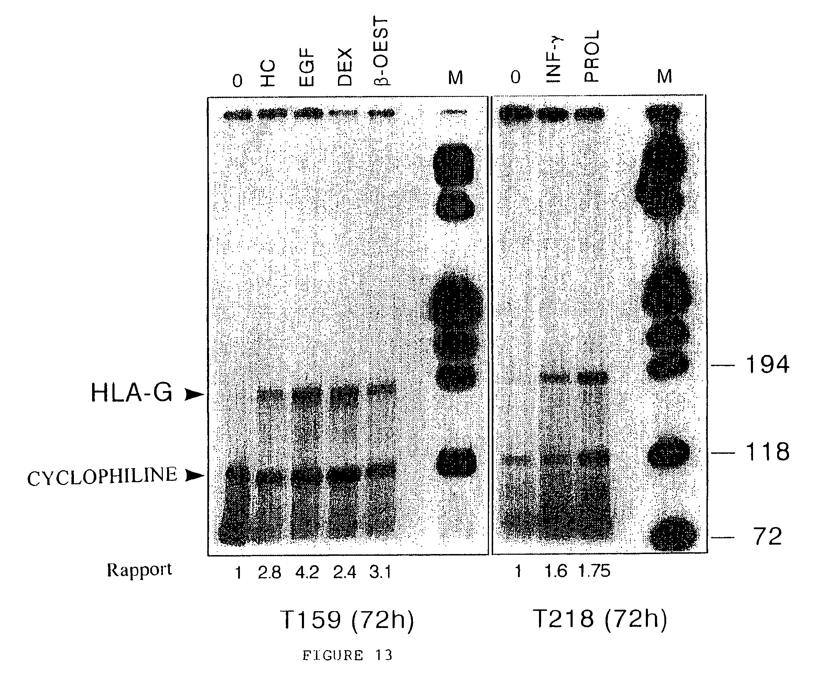

- les figures 12 et 13 illustrent l'augmentation de la transcription des HLA-G, en présence de glucocorticoïdes ou de cytokines.

Il doit être bien entendu, toutefois, que ces exemples sont donnés uniquement à titre d'illustration de l'objet de l'invention, dont ils ne constituent en aucune manière une limitation.

**EXEMPLE 1 : Production des lignées cellulaires contenant les transfectants HLA-G ; leur rôle dans la protection des cellules qui expriment ces molécules à leur surface contre la cytotoxicité des cellules NK.**

**A/ MATÉRIELS ET MÉTHODES**

**1/ Lignées cellulaires :**

La lignée cellulaire érythroleucémique humaine K562 (ATCC) et la lignée cellulaire leucémique T immature (clone YT2C2 à activité NK) sont maintenues dans un milieu RPMI 1640 complémenté avec du sérum de veau foetal à 10%, inactivé par la chaleur, de la L-glutamine 2mM, de la gentamicine à 1 μg/ml et de la fungizone (Sigma, Saint-Quentin, France) et cultivées à 37°C dans un incubateur, humidifié à atmosphère enrichie à 5% en $CO_2$. Les transfectants K562 sont sélectionnés dans un milieu contenant de la généticine à 1 mg/ml (G418 sulfate, Sigma).

La lignée cellulaire humaine de choriocarcinome HLA-G-positive dénommée JEG-3 (ATCC) est cultivée dans un milieu DMEM (Sigma) supplémenté avec du sérum de veau foetal à 10%, inactivé à la chaleur, des antibiotiques et de la L-glutamine 2mM. Les lignées cellulaires ne contiennent pas de mycoplasmes.

**2/ Les vecteurs HLA-G :**

Pour obtenir les transfectants HLA-G1 et HLA-G2, les ADNc correspondant sont insérés dans un vecteur d'expression eucaryote dénommé pRc/RSV (Invitrogen, San Diego, CA), contenant le virus du sarcome de Rous (RSV) comme promoteur et le gène de la néomycine comme marqueur de sélection. La stratégie générale pour la construction du vecteur est illustrée à la Figure 1.

Brièvement, le fragment EcoRI de l'ADNc codant l'HLA-G1 d'une longueur de 1,5-kb est coupé de manière franche en utilisant un fragment Klenow de l'ADN polymérase I et soudé avec le vecteur pRc/RSV digéré par l'enzyme Hind III et déphosphorylé par la phosphatase intestinale de veau (GIBCO-BRL, Life Technologies, Eragny, France). L'orientation correcte de cette construction, dénommée pRc/RSV-G1, est confirmée par digestion enzymatique et séquençage.

Pour obtenir les plasmides recombinants contenant la séquence codant l'HLA-G2, le fragment EcoRI d'ADNc codant la molécule HLA-G1 est inséré dans le site EcoRI du vecteur pGEX-4T-3 (Pharmacia Biotech., Orsay, France). Le fragment d'ADNc codant la molécule HLA-G2 est obtenu par amplification PCR, comme décrit dans KIRSZENBAUM M. et al., Proc. Natl. Acad. Sci. USA, 1994, 91, 4209-4213, en utilisant les amorces spécifiques des HLA-G G.53 et G. 1225. Le fragment obtenu est ensuite digéré par les enzymes de restriction NcoI/BsmI et inséré dans le vecteur pGEX-HLA-G1, à partir duquel le fragment NcoI/BsmI contenant la région codant le domaine α2, a été préalablement retiré (pGEX-HLA-G2). Le plasmide d'expression du HLA-G2 dénommé pRc/RSV-G2 est obtenu après digestion EcoRI du vecteur pGEX-HLA-G2 ; le fragment d'ADNc est coupé de manière franche et inséré dans le vecteur pRc/RSV digéré par l'enzyme de restriction HindIII et qui a été préalablement coupé de manière franche et déphosphorylé.

De manière identique, des vecteurs contenant les isoformes HLA-G3, HLA-G4, HLA-G5 et HLA-G6 peuvent être préparés ; les séquences codant ces différentes isoformes ont été décrites dans la Demande de Brevet Européen n° 0 677 582.

**3/ Lignées cellulaires contenant les transfectants HLA-G :**

Les vecteurs contenant la séquence pRc/RSV-G1 ou la séquence pRc/RSV-G2 ainsi que le vecteur pRc/RSV qui sert de contrôle sont transfectés dans les cellules K562 par électroporation (Bio-Rad Gene Pulsar à 250V, 960 μF). Après deux jours de culture, les cellules sont sélectionnées avec de la généticine à 1 mg/ml dans un milieu RPMI, contenant 10% de sérum de veau foetal. Les cellules contenant les transfectants sont contrôlées par analyse par cytométrie de flux, de manière à confirmer l'expression des molécules HLA de classe I.

Pour obtenir un taux d'expression élevé de HLA-G dans les transfectants K562, les cellules sont isolées par tri cellulaire après immunomarquage (*fluorescence-activated cell sorting ou* FACS, Vantage-Becton Dickinson, Pont de Claix, France), à l'aide d'un anticorps monoclonal spécifique des HLA de classe I.

**4/ Analyses RT-PCR :**

L'ARNm total est extrait à partir de $10^7$ cellules à l'aide du réactif RNA NOW (Biogentex, Inc.) conformément aux recommandations du fabricant. La qualité de l'ARN est vérifiée par électrophorèse sur gel d'agarose à 1,5% dénaturant. Les ADNc sont préparés à partir de 10 μg d'ARN total traités avec de la DNAse I (Boerhinger Mannheim) en utilisant une amorce oligo-$(dT)_{12-18}$ et la transcriptase inverse M-MLV (GIBCO-BRL). Les amplifications RT-PCR sont réalisées en utilisant deux amorces spécifiques des HLA-G, la G.257 (exon 2) et la G. 1225 (3'-UT), comme décrit dans KIRSZENBAUM M. et al. (1994) précité. Dans toutes les amplifications PCR, un mélange de réaction RT sans transcriptase inverse M-MLV (RT⁻) et un mélange PCR sans matrice d'ADNc (blanc), sont utilisés comme contrôles. Une amplification RT-PCR additionnelle de β-actine est réalisée pour pouvoir évaluer la quantité d'ARN dans tous les échantillons. Les produits PCR sont analysés par électrophorèse sur gel d'agarose à 1% et colorés à l'aide de bromure d'éthidium. La spécificité des produits PCR est confirmée par blotting alcalin des fragments dans du NaOH 0,4 N sur des membranes de Nylon (Hybond N+, Amersham, France). L'hybridation est réalisée à l'aide des sondes G. 1200 et G.526 (spécifique de l'exon 3) comme décrit dans KIRSZENBAUM M. et al, 1994 précité. Les filtres sont exposés sur des films Kodak (Biomax) avec des écrans amplificateurs pendant 4 à 16 heures à 1-80°C.

**5/ Immunoprécipitation et électrophorèse SDS-PAGE :**

Les lignées cellulaires JEG-3 et K562 et les lignées transfectantes sont marquées à leur surface avec de la Biotine (Interchim), puis lavées et solubilisées dans un tampon de lyse contenant 1% de Triton X100, 50 m/M de Tris-HCl pH 7,4, 1mM d'EDTA, 150 mM de NaCl et 1 mM de fluorure de phénylméthylsufonyle, 10 μg/ml d'aprotinine et 10 μg/ml de leupeptine (Sigma, Saint-Quentin, France). Les lysats sont centrifugés à 13 000 rpm et préclarifiés par incubation avec une colonne Sépharose-protéine-A CL-4B (Pharmacia Biotechnology, Inc., Upsala, Suède). Les lysats cellulaires préclarifiés sont divisés en deux échantillons et à chacun d'eux sont ajoutées des billes de Sépharose-protéine-A préalablement incubées, soit avec un anticorps monoclonal W6/32 de souris dirigé contre les antigènes de classe I ou un anticorps monoclonal contrôle (UPC-10, IgG2a de souris, Sigma). Après une incubation de deux heures à 4°C, les billes portant les complexes immuns sont lavées et traitées avec un tampon SDS ; l'antigène lié est élué par ébullition des billes. Les protéines précipitées sont analysées par électrophorèse sur SDS-PAGE à 10%. Les gels sont transférés sur des filtres de nitrocellulose, bloqués dans un tampon PBS/Tween 20 à 0,2%/sérum albumine bovine (BSA) à 5% (incubé une nuit à 4°C) et lavés dans un tampon PBS/Tween. Les filtres sont ensuite incubés pendant 40 minutes à température ambiante avec un conjugué peroxydase de raifort-streptavidine. Après un lavage extensif de la membrane, la réaction colorée est réalisée en utilisant le réactif de détection de Western blotting ECL (Amersham, France), après quoi la membrane est exposée à un film Kodak à température ambiante.

**6/ Anticorps monoclonaux et analyses par cytométrie de flux :**

Les anticorps suivants sont utilisés :

W6/32 : IgG2a, anti-chaînes α de HLA de classe I associées à la β2-m (Sigma) ; B1G6 : IgG2a, anti-β2-m (Sigma) ; GL183 : IgG1, anti-p58 (Immunotech, Marseille, France) ; EB6 : IgG1, anti-p58 (Immunotech) ; HP-3B1 : IgG2a, anti-CD94 (Immunotech) ; UCHT-1 : IgG1, anti-CD3 conjugué à du rouge quantum (Sigma) ; 3G8 : IgG1, anti-CD16 conjuguée à de la fluorescéine (Immunotech) ; et B 159 : IgG1 anti-CD56 conjuguée à de la phycoérythrine (Immunotech).

Pour les essais de cytométrie de flux, les cellules sont lavées dans un tampon PBS et marquées avec l'anticorps monoclonal correspondant dans du PBS/Sérum Albumine Bovine 1% pendant 30 minutes à 4°C. Après deux lavages successifs dans un tampon PBS/Sérum Albumine Bovine 1%, les cellules sont soit directement analysées dans un cytomètre de flux (FACS Vantage, Becton Dickinson), si l'anticorps utilisé est conjugué à un fluorochrome, soit marquées avec un fragment F(ab')2 d'immunoglobuline de chèvre anti- IgG de souris conjugué avec de la phycoérythrine (Immunotech) avant l'analyse FACS. Des aliquots contrôles sont marqués avec un anticorps contrôle, pour pouvoir évaluer la liaison non spécifique aux cellules cibles.

**7/ Cellules effectrices NK :**

Les cellules mononucléées du sang périphériques (PBMC) d'adultes sains volontaires (mâles et femelles âgés de 30 à 60 ans) sont obtenues par un gradient de densité Ficoll/histopaque. Les cellules NK sont isolées à partir des PBMC comme suit :

- Les cellules CD3$^+$ sont éliminées en utilisant des billes revêtues d'anticorps anti-CD3 (Dynabeads Dynal, Norvège). Les billes ainsi revêtues sont mélangées avec les cellules mononucléées du sang périphérique dans un rapport 4: 1 billes:cellules CD3$^+$ cibles. Après incubation à 4°C pendant 30 minutes, les cellules cibles ayant formé des complexes avec les billes sont éliminées par passage dans un champ magnétique approprié.
- La fraction dépourvue de cellules CD3$^+$ est alors marquée avec un anticorps monoclonal anti-CD56 conjugué à de la phycoérhythrine, pour sélectionner les cellules CD56$^+$ par tri cellulaire à l'aide d'un cytomètre de flux (FACS).
- Pour contrôler le phénotype des cellules triées (CD56$^+$), un échantillon de cellules est immédiatement marqué avec soit des anticorps monoclonaux anti-CD16 conjugués avec de l'isothiocyanate de fluorescéine (FITC), soit avec un anticorps monoclonal anti-CD3 conjugué avec du rouge quantum, suivi par une analyse fluorescente. La population CD56$^+$ obtenue est viable (> 95%) et présente un phénotype qui correspond aux cellules NK (plus de 90% des cellules NK sont CD3$^-$, CD16$^+$, CD56$^+$).

**9/ Essais de cytotoxicité :**

L'activité cytolytique des cellules mononucléées du sang périphérique, des cellules NK et des cellules YT2C2 (cellules effectrices ou E) à l'encontre des transfectants HLA-G (cellules cibles ou T) est estimée à l'aide de tests de libération pendant 4 heures du chrome 51, dans lesquels les cellules effectrices sont mélangées avec $5.10^3$ de cellules cibles marquées au chrome 51 (100 $\mu$Ci de $^{51}$Cr-chromate de sodium, Amersham, UK), dans différents rapports E/T, dans des plaques de microtitration dont le fond est en forme de U.

Après 4 heures à 37°C dans un incubateur humidifié contenant 5% de $CO_2$, 100 $\mu$l de surnageant sont prélevés pour un comptage par scintillation en phase liquide (Wallac 1450 Microbeta, Pharmacia, France). Le pourcentage de lyse spécifique est calculé comme suit :

$$\text{pourcentage de lyse spécifique} = [\text{cpm dans le puits expérimental} - \text{cpm de libération}$$

$$\text{spontanée})/(\text{cpm de libération maximale} - \text{cpm de libération spontanée})] \times 100.$$

La libération spontanée est déterminée par incubation des cellules cibles (T) marquées avec le milieu. La libération maximale est déterminée par solubilisation des cellules cibles dans de l'HCl 0,1 M. Dans toutes les expériences, la libération spontanée est inférieure à 10% par rapport à la libération maximale. Les résultats sont présentés comme des moyennes de trois échantillons. Dans les expériences dans lesquelles les anticorps monoclonaux sont utilisés pour bloquer l'interaction HLA-G-NK, les cellules cibles sont incubées avec l'anticorps monoclonal correspondant, puis lavées et incubées avec un anticorps F(ab')$_2$ de chèvre anti-souris (Jackson Immunoresearch, USA) pour éviter la cytotoxicité cellulaire dépendante des anticorps (ADCC) par interaction des récepteurs pour le fragment Fc des immunoglulines, exprimés sur les cellules NK avec le premier anticorps utilisé. Les toxicités des anticorps monoclonaux sont également vérifiées dans chaque essai et sont toujours inférieures à 3%.

**B/ RESULTATS :**

**1/ Production de transfectants stables exprimant la molécule HLA-G1 ou la molécule HLA-G2:**

L'analyse des séquences d'ADNc d'HLA-G1 et d'HLA-G2 transfectées démontre que l'ADNc d'HLA-G1 ne contient pas les deux codons GAC correspondant aux positions en acides aminés 247 et 248 (ELLIS et al., J. Immunol, 1990, 144, 731-735), mais seulement un seul codon GAC, correspondant à la position en acides aminés 247 (PAZMANY L. et al., Science, 1996, 274, 792-795). La séquence d'ADNc codant l'HLA-G2, obtenue par amplification RT-PCR à l'aide des sondes G.53 et G.1225, présente une jonction entre les exons 2 et 4, en raison de l'absence de l'exon 3 dans cette forme d'ARNm épissée.

La caractérisation des transfectants est réalisée par une analyse en Southern blot des produits de la RT-PCR obtenus en utilisant les amorces G.257 et G.1225 avec la lignée cellulaire parentale K562, les transfectants et les cellules JEG-3. Les résultats sont illustrés à la Figure 2. L'hybridation des produits de la RT-PCR obtenus, avec la sonde G. 1200, révèle une bande de 0,98 kb pour le transfectant K562-HLA-G1 correspondant à l'ARNm d'HLA-G1 et une bande de 0,71 kb correspondant à la forme épissée alternative, qui ne comprend pas l'exon 3 (KIRSZENBAUM M. et al., Hum. Immunol., 1995, 43, 237-241). Avec les cellules JEG-3, trois bandes sont observées à 0,98 kb (HLA-G1), 0,7 kb (HLA-G2 et HLA-G4) et 0,43 kb (HLA-G3) (MOREAU P. et al., Hum. Immunol., 1997, 52, 41-46). Par contraste, aucune bande HLA-G n'est détectée dans la lignée cellulaire parentale K562 ou dans la lignée transfectante contrôle K562-pRc/RSV. L'hybridation des mêmes produits de la RT-PCR avec la sonde spécifique de l'exon 3 G.526 révèle une seule bande dans les cellules transfectées HLA-G1 tandis qu'aucune hybridation n'est observée dans les transfectants HLA-G2, en raison de l'absence de l'exon 3 dans la forme HLA-G2 (Figure 2). L'amplification RT-PCR du gène de la β-actine, constitutivement exprimée, démontre que l'absence de bandes HLA-G dans les contrôles K562 n'est pas due à l'absence d'ARN (Figure 2).

**2/ Immunoprécipitation and analyse SDS-PAGE des protéines de surface marquées :**

L'immunoprécipitation des protéines HLA avec l'anticorps monoclonal W6/32 est réalisée, afin d'établir si les transfections permettent d'obtenir une expression spécifique des protéines HLA-G à la surface. Comme illustré à la Figure 3, le Western blot correspondant révèle que l'anticorps monoclonal W6/32 immunoprécipite une molécule de 39-kDa dans les cellules K562 qui expriment la protéine HLA-G1 (piste 6) et dans les cellules JEG-3 (piste 10). Pour les transfectants HLA-G2, l'immunoprécipitation à l'aide de l'anticorps W6/32 montre une bande correspondant à la taille de la protéine G2. Aucune molécule du système HLA (molécule HLA classique de classe I, HLA-G1 ou HLA-G2) n'est trouvée ni dans les cellules parentales K562, ni dans les cellules contrôles K562 pRc-RSV.

**3/ Analyse par cytométrie de flux des transfectants HLA-G :**

Pour déterminer le taux d'expression d'HLA-G à la surface des cellules, l'analyse des profils en cytométrie de flux des différentes cellules est réalisée ; les résultats sont illustrés à la Figure 4 et concernent les différents lignées étudiées : la lignée cellulaire parentale K562, la lignée contrôle comprenant le plasmide pRc/RSV, les transfectants HLA-G1 et HLA-G2 ainsi que la lignée cellulaire JEG-3 de choriocarcinome positive pour l'expression du HLA-G. Les résultats observés montrent que la lignée cellulaire parentale K562 n'exprime aucun taux détectable de molécule de surface HLA de classe I (estimation à l'aide de l'anticorps pan-HLA de classe I W6/32 et à l'aide de l'anticorps anti-β2m B1G6). Aucune molécule HLA de classe I n'est détectée dans la lignée cellulaire K562 transfectée avec le vecteur vide pRc/RSV. Le transfectant HLA-G1 est marqué par les anticorps W6/32 et B1G6 à peu près de la même manière que ce qui est obtenu avec les cellules JEG-3. Le transfectant HLA-G2 est également marqué par l'anticorps W6/32 ainsi que l'anticorps B1G6. Ce résultat montre que l'isoforme HLA-G2 liée à la membrane est bien présente à la surface des cellules, associée avec la β2-microglobuline, de la même manière que les molécules HLA de classe I classiques.

**4/ Résistance des transfectants HLA-G1 et HLA-G2 à la cytolyse par les cellules NK :**

Les transfectants HLA-G1 et HLA-G2 sont testés parallèlement à la lignée parentale correspondante pour leur sensibilité aux cellules NK. 20 expériences ont été effectuées, chacune enregistrée avec des cellules mononucléées de différents donneurs. La Figure 5 montre que l'activité lytique des cellules NK, présentes dans les cellules mononucléées du sang périphérique isolé à partir de trois adultes donneurs sains, est inhibée aussi bien par les transfectants K562 HLA-G1 que par les transfectants K562 HLA-G2, tandis que la lignée cellulaire contrôle K562 pRC/RSV reste sensible à l'activité lytique des cellules NK.

Bien que le taux absolu de lyse varie d'un donneur à l'autre, comme illustré à la Figure 5 pour les donneurs désignés 4010, 845 et 813 qui présentent respectivement des taux de lyse élevés, moyens et bas, la sensibilité des cellules exprimant la molécule HLA-G1 et la molécule HLA-G2 est réduite d'au moins 70%. Ces résultats sont confirmés lorsque l'on utilise des cellules polyclonales NK CD3[-], CD16[+], CD56[+], obtenues à partir de cellules mononucléées de sang périphérique de trois autres donneurs comme cellules effectrices (Figure 6). En outre, aucune différence n'est observée entre les donneurs mâles et femelles.

Afin de montrer que l'inhibition de l'activité lytique des cellules NK est due effectivement à la présence des molécules HLA-G1 et HLA-G2 à la surface des cellules cibles, des tests de cytotoxicité sont réalisés en présence de cellules cibles préincubées soit avec l'anticorps W6/32 qui reconnaît les molécules HLA-G1 et HLA-G2, soit à l'aide d'un anticorps contrôle. L'addition d'anticorps monoclonal W6/32 lève l'inhibition de l'activité des cellules NK, due à la présence des molécules HLA-G1 et HLA-G2 et, par conséquent, restaure l'activité lytique des cellules NK sur les cellules cibles transfectées (Figures 7a et 7b). Dans la mesure où le mécanisme de la cytotoxicité cellulaire dépendante des anticorps (ADCC) pourrait être impliqué dans cette réaction par l'interaction avec les récepteurs au fragment Fc des cellules NK, le fragment Fc de l'anticorps W6/32 est bloqué par un fragment $F(ab')_2$ d'anticorps de chèvre anti-IgG de souris.

**5/ Inhibition de la lyse induite par le clone YT2C2 à activité NK, par les transfectants HLA-G1 et HLA-G2 :**

Le clone YT2C2 correspondant à des cellules T immatures leucémiques est utilisé comme effecteur contre les transfectants HLA-G. La Figure 8 montre qu'aussi bien les transfectants HLA-G1 que les transfectants HLA-G2 abolissent la lyse induite par le clone YT2C2, tandis que les lignées cellulaires contrôles ne le font pas. Cependant, contrairement à ce qui est observé avec les cellules mononucléées du sang périphérique, l'inhibition de la lyse induite par le clone YT2C2 n'est pas levée par l'utilisation de cellules cibles transfectées traitées par l'anticorps monoclonal W6/32 (Figure 7c), suggérant que l'épitope reconnu par l'anticorps monoclonal sur la molécule HLA-G n'est pas impliqué dans la reconnaissance de la molécule HLA-G par le récepteur qui est présent sur les cellules YT2C2.

Une analyse par immunofluorescence indirecte est réalisée, de manière à vérifier le phénotype des cellules YT2C2. Pour ce faire, les anticorps monoclonaux EB6 et GL183, qui reconnaissent respectivement les récepteurs NKIR1 et NKIR2, exprimés à la surface de sous-groupes de cellules NK, ainsi que l'anticorps HP-3B 1, qui reconnaît le récepteur CD94 et qui est exprimé sur la plupart des cellules NK, ont été utilisés. Ces récepteurs sont impliqués dans la reconnaissance des molécules HLA-A, -B ou -C par les cellules NK (COLONNA et al., Science, 1995, 268; 405-408 et MORETTA et al., J. Exp. Med, 1994, 180, 545-555). La Figure 8 montre que les cellules YT2C2 présentent le phénotype négatif suivant : EB6[-], GL183[-] et HP3B1[-]. Ces anticorps monoclonaux ont été préalablement testés par immunofluorescence indirecte et marquent positivement 3 à 10% des cellules mononucléées du sang périphérique des donneurs adultes sains. Ces différents résultats montrent que, en plus des récepteurs dénommés NKIR1 et NKIR2, qui ont été récemment décrits comme reconnaissant HLA-G (PAZMANY et al. (1996) précité), le clone YT2C2 exprime un nouveau récepteur capable de lier les molécules HLA-G et d'induire une protection contre la lyse par les cellules YT2C2.

L'ensemble des résultats montre que les antigènes HLA-G jouent un rôle dans la protection des cellules qui expriment ces molécules à leur surface contre la cytotoxicité induite par les cellules NK. Contrairement aux travaux de l'art antérieur (CHUMBLEY et al., Cell Immunol., 1994, 155, 312-322 ; DENIZ et al., J. Immunol., 1994, 152, 4255-4261 et PAZMANY L. et al. (1996) précité), dans lesquels il a été proposé de transfecter l'ADN génomique codant la molécule HLA-G dans une cellule cible, pour étudier ses effets sur l'activité lytique de clone NK, les Inventeurs ont trouvé qu'en évaluant séparément les isoformes HLA-G1 et HLA-G2, dans des conditions physiologiques face à des cellules NK polyclonales obtenues à partir de donneurs sains adultes, il était possible de manière surprenante : (i) d'exprimer les molécules HLA-G1 et HLA-G2, à la surface des cellules cibles et (ii) de montrer que de telles cellules cibles ainsi modifiées étaient capables d'inhiber l'activité lytique desdites cellules NK.

Le Tableau I ci-après démontre qu'aussi bien l'isoforme HLA-G1 et que l'isoforme HLA-G2 liées à la membrane cellulaire inhibent fortement la lyse induite par les cellules NK.

Tableau 1

| Lignée cellulaire | transcrit mRNA | | expression membranaires des HLA de classe I | Résistance à la lyse NK |
|---|---|---|---|---|
| | HLA-G1 | HLA-G2 | W6/32 | |
| Non transfectée K562 | - | - | - | - |
| Transfectées K562-pRc/RSV | - | - | - | - |
| K562-HLA-G1 | + | - | + | + |
| K562-HLA-G2 | - | + | + | + |

Cette inhibition est levée lorsque les cellules transfectées qui expriment la molécule HLA-G1 ou la molécule HLA-G2 sont incubées avec l'anticorps monoclonal pan-classe I, indiquant que l'inhibition de la lyse induite par les cellules NK est due à la présence des molécules HLA-G à la surface des cellules cibles transfectées K562 (Figure 7). Le fait que l'activité lytique NK soit inhibée chez tous les donneurs testés par les cellules cibles HLA-G positives, confirme que la molécule HLA-G pourrait être le ligand public du récepteur inhibiteur NK présent chez tous les individus (NKIR) et montre l'intérêt de l'obtention de cellules présentant un taux important et prédictible d'expression de molécules HLA-G.

De manière surprenante, il ressort de ces résultats que le domaine $\alpha$1 commun à ces deux isoformes devrait être considéré comme jouant un rôle important dans la protection contre les cellules NK. Ceci peut être soutenu par le fait que les séquences en amino acides des molécules HLA de classe I nécessaires pour l'interaction avec le récepteur KIR sont localisées dans le domaine α1 (COLONNA et al., Proc. Natl. Acad. Sci., 1992, 89, 7983-7985 et MANDELBOIM et al., J. Exp. Med., 1996, 184, 913-922). L'analyse séquentielle montre que le domaine α1 des molécules HLA-G présente des amino acides qui ont été antérieurement décrits comme induisant une résistance contre la cytolyse induite par les cellules NK dans les allèles protecteurs HLA-A, HLA-B et HLA-C (PAZMANY et al. précité). De manière surprenante, les Inventeurs ont trouvé maintenant que la molécule HLA-G partage des caractéristiques structurelles avec les allèles protecteurs HLA-A3, A11, Aw68, Aw69, B7 et B27. En particulier, la position 74 dans le domaine α1 de l'HLA-G est occupé par un résidu Asp, associé avec l'induction d'une résistance aux cellules NK.

En outre, les Inventeurs ont trouvé, également de manière surprenante, que les cellules T leucémiques YT2C2 n'induisaient plus de lyse en présence de transfectants exprimant la molécule HLA-G1 ou la molécule HLA-G2, bien qu'aucun des récepteurs KIR capables de lier l'HLA-G (récepteur p58.1, récepteur p58.2 et CD94) ne soient présents sur les cellules YT2C2 (Figure 8). Ces résultats suggèrent qu'un nouveau récepteur distinct de ceux déjà décrits est impliqué dans la reconnaissance des molécules HLA-G 1 et HLA-G2.

**EXEMPLE 2 : Mise en évidence de l'activité inhibitrice des isoformes d'HLA-G dans la réponse proliférative allogénique**

On réalise une culture mixte de cellules mononucléées provenant du sang périphérique de deux donneurs volontaires présentant des antigènes HLA de classe II différents. Les cellules d'un donneur dites stimulantes, ont été irradiées à 3000 rads pour éviter leur prolifération. Seules les cellules répondantes de l'autre individu peuvent proliférer.

On introduit dans ces cultures, soit des cellules K562 contenant le plasmide pRc/RSV, à titre de contrôle (pas d'expression d'antigène du CMH), soit des cellules K562 transfectées et exprimant à leur surface, une isoforme d'HLA-G, par exemple l'isoforme HLA-G 1 (insertion du plasmide pRc/RSV-G1). Les cellules K562 ont été irradiées, afin d'éviter toute prolifération.

La figure 9 illustre les résultats obtenus : on observe, sur quatre donneurs différents, une inhibition de la réponse proliférative allogénique, de l'ordre de 50 à 67 %.

**EXEMPLE 3 : Inhibition de l'activité NK par la protéine HLA-G5**

Exprimée par une cellule eucaryote selon l'invention ou directement injectée, la protéine HLA-G5 inhibe, comme les autres isoformes d'HLA-G, l'activité cytolytique des cellules NK.

Cette inhibition est dose-dépendante, comme le montrent les figures 10 et 11, qui sont le résultat d'essais réalisés avec une protéine de fusion GST-HLA-G5 exprimée chez *E. coli*. Cette protéine de fusion est purifiée sur une colonne glutathion-Sépharose (Pharmacia Biotech) ou par chromatographie d'affinité avec des anticorps anti-HLA ou des anticorps anti-GST, après extraction à partir des cultures bactériennes (centrifugation, récupération du culot dans un tampon STE, lyse des cellules avec du N-laurylsarcosine à 1,5 %, clarification du lysat par centrifugation et solubilisation du surnageant par du triton X-100 à 2 %).

Les essais de cytotoxicité sont réalisés comme précisé ci-dessus, en utilisant des cellules K562, sensibles aux cellules NK, comme témoin positif.

14 expériences, réalisées sur des cellules mononucléées de différents donneurs sont réalisées en présence de GST-HLA-G5 ou de Sépharose-GST-HLA-G5.

La figure 10 montre que l'activité lytique des PBMC, isolées de trois donneurs sains adultes, est inhibée par des billes de Sépharose-GST-HLA-G5 ; la figure 11 confirme que la protéine soluble présente la même activité inhibitrice sur la lyse induite par les cellules NK, que les autres isoformes.

**EXEMPLE 4 : Facteurs intervenant dans la stimulation de la transcription des différentes isoformes d'HLA-G.**

**Test de protection de la RNase**

**A/ MATERIEL ET METHODE**

- Radiosondes :

des sondes ARNc simple-brins radiomarquées sont synthétisées à l'aide du kit de transcription MAXIScript® (Ambion) en présence de T7 ARN polymérase et de 5 μl de (α-$^{32}$P) CTP (Amersham), conformément aux indications du fabricant.

Une matrice cyclophiline est fournie par le fabricant (Ambion). Une matrice HLA-G est obtenue par amplification en chaîne (PCR) d'un fragment d'HLA-G génomique correspondant à une partie de la région non traduite (région 3' UT). Les amorces utilisées sont les suivantes :

amorce sens (G.1089F) : 5'-CCCTTTGTGACTTCAAGAAC
amorce anti-sens (T7G.1250R) : 5'-**GGATCCTAATACGACT CACTATAGGGAGG**TTATAGCTCAGTGGCCCAC.

Cette amorce contient une séquence qui permet de générer un promoteur T7 pendant la PCR (partie en gras).

Les sondes sont purifiées par électrophorèse sur un gel acrylamide à 6 % + urée 8 M environ 1 h à 200 V.

Après l'électrophorèse, le gel est exposé à un film sensible aux rayons X.

La zone du gel contenant le transcrit marqué complet est immergée dans 350 $\mu$l de tampon d'élution de sonde (kit Ambion).

- Méthode :

La protection des transcrits HLA-G est réalisée conformément au kit HybSpeed® RPA kit (Ambion), conformément aux recommandations du fabricant.

5 $\mu$g d'ARN total sont isolés avec le réactif RNA NOW (Ozyme) selon les recommandations du fabricant ; $5.10^5$ cpm de ribosonde HLA-G et $5.10^5$ cpm de ribosonde cyclophiline sont hybridés pendant 10 min dans un tampon d'hybridation HybSpeed®.

Un mélange d'ARNase A/T à une concentration finale de 1/100ème est ajouté pendant 30 secondes. Ces fragments protégés et radiomarqués sont précipités et séparés sur un gel acrylamide à 5 % : gel bis-acrylamide (19:1) (100 V).

Le gel est séché et exposé (Biorad) pour quantifier le signal HLA-G.

Les valeurs sont comparées aux signaux obtenus avec la cyclophiline.

## B/RESULTATS

Différentes hormones et cytokines augmentent la transcription de la molécule HLA-G. Les figures 12 et 13 illustrent les résultats obtenus après incubation, pendant 72 heures, de trophoblastes ou de cellules eucaryotes exprimant au moins une isoforme d'HLA-G avec les différents facteurs suivants : interleukine 10, interleukine 1β, interféron y, TGF-β, EGF, prolactine, β oestradiol, hydrocortisone et dexaméthasone.

Ces figures illustrent l'augmentation de la transcription d'ARNm, par la technique de RPP.

Ces figures 12 et 13 montrent en particulier que le rapport d'augmentation, vis-à-vis des trophoblastes, en présence de milieux de culture et sans aucune stimulation, est de 2x à 7,3x, particulièrement pour IL 10, IL 1β, TGF β et les glucocorticoïdes.

Ces résultats montrent en particulier l'intérêt des produits selon l'invention dans la prévention des avortements spontanés à répétition.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

**Revendications**

1. Utilisation d'une cellule eucaryote modifiée par au moins une copie d'un ADNc codant une isoforme de la molécule HLA-G sélectionnée dans le groupe constitué par les isoformes HLA-G1, HLA-G2, HLA-G3, HLA-G4, HLA-G5 et HLA-G6, ladite cellule exprimant ladite isoforme d'HLA-G membranaire ou sécrétée, pour l'obtention d'une composition destinée à inhiber la réponse allogénique primaire, dans la prévention du rejet de greffe.

2. Utilisation selon la revendication 1, **caractérisée en ce que** ladite cellule est une cellule hépatique, une cellule rénale ou une cellule souche hématopoïétique.

3. Utilisation selon la revendication 1 ou la revendication 2, **caractérisée en ce que** ladite cellule est une cellule humaine.

4. Utilisation selon l'une quelconque des revendications 1 à 3, **caractérisée en ce que** la dite composition est destinée à être transplantée dans un tissu ou un organe à greffer.

5. Utilisation selon la revendication 1 ou la revendication 2, **caractérisée en ce que** ladite cellule est incluse dans un tissu ou un organe d'un mammifère transgénique non-humain comprenant ledit ADNc codant une isoforme de la molécule HLA-G.

6. Utilisation selon l'une quelconque des revendications 1, 2 et 5, **caractérisée en ce que** ladite composition est un tissu ou organe destiné à la xénotransplantation.

7. Utilisation selon l'une quelconque des revendications 1 à 6, **caractérisée en ce que** ladite cellule exprime l'isoforme soluble HLA-G5.

8. Produit contenant au moins une cellule eucaryote modifiée par au moins une copie d'un ADNc codant une isoforme de la molécule HLA-G sélectionnée dans le groupe constitué par les isoformes HLA-G1, HLA-G2, HLA-G3, HLA-G4, HLA-G5 et HLA-G6 et un facteur de stimulation de l'expression d'HLA-G sélectionné dans le groupe constitué par les corticoïdes et les cytokines, en tant que préparation combinée, pour une utilisation simultanée, séparée ou étalée dans le temps, dans la prévention du rejet de greffe.

9. Produit selon la revendication 8, **caractérisé en ce que** ledit facteur de stimulation est sélectionné dans le groupe constitué par l'IL-10, l'IL1-β, le TGF-β, l'hydrocortisone et la dexaméthasone.

10. Cellule eucaryote modifiée par au moins une copie d'un ADNc codant une isoforme de la molécule HLA-G, **caractérisée en ce qu'**elle est modifiée par un vecteur d'expression comprenant au moins une copie d'un ADNc codant une isoforme de la molécule HLA-G, sélectionnée dans le groupe constitué par HLA-G2, HLA-G3, HLA-G4, HLA-G5 et HLA-G6.

11. Cellule eucaryote modifiée par au moins une copie d'un ADNc codant une isoforme de la molécule HLA-G, **caractérisée en ce qu'**elle est modifiée par un vecteur d'expression comprenant une origine de réplication appropriée, un marqueur de sélection, le promoteur viral RSV et une copie d'un ADNc codant une isoforme de la molécule HLA-G, sélectionnée dans le groupe constitué par HLA-G1, HLA-G2, HLA-G3, HLA-G4, HLA-G5 et HLA-G6.

**12.** Mammifère non-humain transgénique comprenant des cellules exprimant une molécule HLA-G, **caractérisé en ce qu'**il est modifié par un vecteur d'expression comprenant au moins une copie d'un ADNc codant une isoforme de la molécule HLA-G, sélectionnée dans le groupe constitué par HLA-G2, HLA-G3, HLA-G4, HLA-G5 et HLA-G6 ou un vecteur d'expression comprenant une origine de réplication appropriée, un marqueur de sélection, le promoteur viral RSV et une copie d'un ADNc codant une isoforme de la molécule HLA-G, sélectionnée dans le groupe constitué par HLA-G1, HLA-G2, HLA-G3, HLA-G4, HLA-G5 et HLA-G6.

**13.** Tissu ou organe non-humain transgénique isolé, provenant d'un mammifère comprenant au moins une copie d'un ADNc codant pour une isoforme de la molécule d'HLA-G.

**14.** Cellule eucaryote modifiée par au moins une copie d'un ADNc codant une isoforme de la molécule HLA-G , comme médicament pour la prévention du rejet de greffe.

**15.** Cellule d'un mammifère non-humain transgénique comprenant au moins une copie d'un ADNc codant une isoforme d'une molécule HLA-G , un vecteur d'expression comprenant au moins une copie d'un ADNc codant une isoforme de la molécule HLA-G, sélectionnée dans le groupe constitué par HLA-G2, HLA-G3, HLA-G4, HLA-G5 et HLA-G6 ou un vecteur d'expression comprenant une origine de réplication appropriée, un marqueur de sélection, le promoteur viral RSV et une copie d'un ADNc codant une isoforme de la molécule HLA-G, sélectionnée dans le groupe constitué par HLA-G1, HLA-G2, HLA-G3, HLA-G4, HLA-G5 et HLA-G6, pour une utilisation comme xénogreffe, dans la transplantation d'organe.

**Claims**

**1.** Use of a eukaryotic cell modified by at least one copy of a cDNA encoding an isoform of the molecule HLA-G selected from among the isoforms HLA-G1, HLA-G2, HLA-G3, HLA-G4, HLA-G5 and HLA-G6, said cell expressing said membranal or secreted HLA-G isoform, for obtaining a composition intended to inhibit the primary allogenic response, in the prevention of graft rejection.

**2.** Use according to claim 1, **characterised in that** said cell is a hepatic cell, a renal cell or a haematopoietic stem cell.

**3.** Use according to claim 1 or claim 2, **characterised in that** said cell is a human cell.

**4.** Use according to any one of claims 1 to 3, **characterised in that** said composition is intended to be transplanted into a tissue or organ that is to be grafted.

**5.** Use according to claim 1 or claim 2, **characterised in that** said cell is included in a tissue or organ of a non-human transgenic mammal comprising said cDNA encoding an isoform of the molecule HLA-G.

**6.** Use according to any one of claims 1, 2 and 5, **characterised in that** said composition is a tissue or organ intended for xenotransplantation.

**7.** Use according to any one of claims 1 to 6, **characterised in that** said cell expresses the soluble isoform HLA-G5.

**8.** Product containing at least one eukaryotic cell modified by at least one copy of a cDNA encoding an isoform of the molecule HLA-G selected from among the isoforms HLA-G1, HLA-G2, HLA-G3, HLA-G4, HLA-G5 and HLA-G6 and a factor for stimulating HLA-G expression selected from among the corticoids and cytokines, as a combined preparation, for simultaneous, separate or staggered over time use in the prevention of graft rejection.

**9.** Product according to claim 8, **characterised in that** said stimulation factor is selected from among 1L-10, IL1-β, TGF-β, hydrocortisone and dexamethasone.

**10.** Eukaryotic cell modified by at least one copy of a cDNA encoding an isoform of the molecule HLA-G, **characterised in that** it is modified by an expression vector comprising at least one copy of a cDNA encoding an isoform of the molecule HLA-G, selected from among HLA-G2, HLA-G3, HLA-G4, HLA-G5 and HLA-G6.

**11.** Eukaryotic cell modified by at least one copy of a cDNA encoding an isoform of the molecule HLA-G, **characterised in that** it is modified by an expression vector comprising a suitable origin of replication, a selection marker, the viral promoter RSV and a copy of a cDNA encoding an isoform of the molecule HLA-G, selected from among HLA-G1, HLA-G2, HLA-G3, HLA-G4, HLA-G5 and HLA-G6.

12. Transgenic non-human mammal comprising cells expressing a molecule HLA-G, **characterised in that** it is modified by an expression vector comprising at least one copy of a cDNA encoding an isoform of the molecule HLA-G, selected from among HLA-G2, HLA-G3, HLA-G4, HLA-G5 and HLA-G6 or an expression vector comprising a suitable origin of replication, a selection marker, the viral promoter RSV and a copy of a cDNA encoding an isoform of the molecule HLA-G, selected from among HLA-G1, HLA-G2, HLA-G3, HLA-G4, HLA-G5 and HLA-G6.

13. Isolated transgenic non-human tissue or organ, coming from a mammal comprising at least one copy of a cDNA encoding an isoform of the molecule HLA-G.

14. Eukaryotic cell modified by at least one copy of a cDNA encoding an isoform of the molecule HLA-G, as a medicament for preventing graft rejection.

15. Cell of a transgenic non-human mammal comprising at least one copy of a cDNA encoding an isoform of a molecule HLA-G, an expression vector comprising at least one copy of a cDNA encoding an isoform of the molecule HLA-G, selected from among HLA-G2, HLA-G3, HLA-G4, HLA-G5 and HLA-G6 or an expression vector comprising a suitable origin of replication, a selection marker, the viral promoter RSV and a copy of a cDNA encoding an isoform of the molecule HLA-G, selected from among HLA-G1, HLA-G2, HLA-G3, HLA-G4, HLA-G5 and HLA-G6, for use as a xenograft, in organ transplantation.

**Patentansprüche**

1. Verwendung einer eukaryotischen Zellen, die durch mindestens eine Kopie einer cDNA modifiziert ist, die für eine Isoform des HLA-G-Moleküls codiert, ausgewählt aus der Gruppe bestehend aus den Isoformen HLA-G1, HLA-G2, HLA-G3, HLA-G4, HLA-G5 und HLA-G6, wobei die Zelle die membrangebundene oder die sezernierte Isoform des HLA-G exprimiert, zur Bereitstellung einer Zusammensetzung für die Hemmung der primären allogenen Antwort, bei der Vorbeugung der Abstoßung von Transplantaten.

2. Die Verwendung gemäß Anspruch 1, **dadurch gekennzeichnet, dass** die Zelle eine Leberzelle, eine Nierenzelle oder eine hämatopoietische Stammzelle ist.

3. Die Verwendung gemäß Anspruch 1 oder 2, **dadurch gekennzeichnet, dass** die Zelle eine menschliche Zelle ist.

4. Die Verwendung nach irgendeinem der Ansprüche 1 bis 3, **dadurch gekennzeichnet, dass** die Zusammensetzung bestimmt ist in ein Gewebe oder in ein Organtransplantat übertragen zu werden.

5. Die Verwendung nach Anspruch 1 oder 2, **dadurch gekennzeichnet, dass** die Zelle in einem Gewebe oder in einem Organ eines nicht-menschlichen transgenen Säugetiers enthalten ist, das die für eine Isoform des HLA-G-Moleküls codierende cDNA enthält.

6. Die Verwendung nach Anspruch 1, 2 und 5, **dadurch gekennzeichnet, dass** die Zusammensetzung ein Gewebe oder ein Organ ist, welches für die Xenotransplantation bestimmt ist.

7. Die Verwendung nach irgendeinem der Ansprüche 1 bis 6, **dadurch gekennzeichnet, dass** die Zelle die lösliche Isoform HLA-G5 exprimiert.

8. Produkt enthaltend mindestens eine eukaryotische Zelle, die durch mindestens eine Kopie einer cDNA modifiziert ist, die für eine Isoform des HLA-G-Moleküls codiert, ausgewählt aus der Gruppe bestehend aus den Isoformen HLA-G1, HLA-G2, HLA-G3, HLA-G4, HLA-G5 und HLA-G6, und einen Faktor, der die Expression der HLA-G-Moleküle fördert, ausgewählt aus der Gruppe bestehend aus Kortikoiden (Kortikosteroiden) und Cytokinen, als Kombinantionspräparat für die gleichzeitige, getrennte oder zeitlich gestaffelte Anwendung bei der Vorbeugung der Abstoßung von Transplantaten.

9. Das Produkt gemäß Anspruch 8, **dadurch gekennzeichnet, dass** der Stimulationsfaktor ausgewählt ist aus der Gruppe bestehend aus IL-10, IL1-β, TGF-β, Hydrocortison und Dexamethason.

10. Eukaryotische Zelle, die durch mindestens eine Kopie einer cDNA modifiziert ist, die für eine Isoform des HLA-G-Moleküls codiert, **dadurch gekennzeichnet, dass** die Zelle mit einem Expressionsvektor transformiert ist, der mindestens eine Kopie einer cDNA enthält, die für eine Isoform des HLA-G-Moleküls codiert ausgewählt aus der Gruppe bestehend aus HLA-G2, HLA-G3, HLA-G4, HLA-G5 und HLA-G6.

11. Eukaryotische Zelle, die durch mindestens eine Kopie einer cDNA modifiziert ist, die für eine Isoform des HLA-G-Moleküls codiert, **dadurch gekennzeichnet, dass** die Zelle mit einem Expressionsvektor transformiert ist, der einen geeigneten Replikationsursprung, einen Selektionsmarker, den viralen RSV-Promotor und eine Kopie einer cDNA enthält, die für eine Isoform des HLA-G-Moleküls codiert ausgewählt aus der Gruppe bestehend aus HLA-G1, HLA-G2, HLA-G3, HLA-G4, HLA-G5 und HLA-G6.

12. Nicht-menschliches transgenes Säugetier, enthaltend Zellen, die HLA-G-Moleküle exprimieren, **dadurch gekennzeichnet, dass** es mit einem Expressionsvektor transformiert ist, der mindestens eine Kopie einer cDNA enthält, die für eine Isoform des HLA-G-Moleküls codiert ausgewählt aus der Gruppe bestehend aus HLA-G2, HLA-G3, HLA-G4, HLA-G5 und HLA-G6 oder mit einem Expressionsvektor transformiert ist, der einen geeigneten Replikationsursprung, einen Selektionsmarker, den viralen RSV-Promotor und eine Kopie einer cDNA enthält, die für eine Isoform des HLA-G-Moleküls codiert ausgewählt aus der Gruppe bestehend aus HLA-G1, HLA-G2, HLA-G3, HLA-G4, HLA-G5 und HLA-G6.

13. Nicht-menschliches isoliertes transgenes Gewebe oder Organ aus einem Säugetier stammend, enthaltend mindestens eine Kopie einer cDNA, die für eine Isoform des HLA-G-Moleküls codiert.

14. Eukaryotische Zelle, die durch mindestens eine Kopie einer cDNA modifiziert ist, die für eine Isoform des HLA-G-Moleküls codiert, als Arzneimittel für die Vermeidung der Abstoßung von Transplantaten.

15. Nicht-menschliche transgene Säugetierzelle enthaltend mindestens eine Kopie einer cDNA, die für eine Isoform des HLA-G-Moleküls codiert, einen Expressionsvektor, der mindestens eine Kopie einer cDNA enthält, die für eine Isoform des HLA-G-Moleküls codiert ausgewählt aus der Gruppe bestehend aus HLA-G2, HLA-G3, HLA-G4, HLA-G5 und HLA-G6 oder einen Expressionsvektor, der einen geeigneten Replikationsursprung, einen Selektionsmarker, den viralen RSV-Promotor und eine Kopie einer cDNA enthält, die für eine Isoform des HLA-G-Moleküls codiert ausgewählt aus der Gruppe bestehend aus HLA-G1, HLA-G2, HLA-G3, HLA-G4, HLA-G5 und HLA-G6 für die Verwendung als Xenotransplantat bei einer Organtransplantation.

EP 0 917 538 B1

FIGURE 1

FIGURE 2

FIGURE 3

FIGURE 4

FIGURE 5

FIGURE 6

FIGURE 7

26

FIGURE 8

# RÔLE DE LA MOLECULE HLA-G DANS LA REPONSE PROLIFERATIVE ALLOGENIQUE

K562-RSV

K562-G1 irradiées

EP 0 917 538 B1

|  | 747 | 876 | 476 | 561 |
|---|---|---|---|---|
| **Cellule répondante** | Dr 7/10<br>Dq 2/5 | Dr 13/6<br>Dq 6/6 | Dr 4/7<br>Dq 2/3 | Dr 6/5<br>Dq 3 |
| **Cellule stimulante irradiée** | 219<br>Dr 1/3<br>Dq 5/2 | 219<br>Dr 1/3<br>Dq 5/2 | 219<br>Dr 1/3<br>Dq 5/2 | 219<br>Dr 1/3<br>Dq 5/2 |

FIGURE 9

FIGURE 10

FIGURE 11

FIGURE 12

FIGURE 13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0677582 A

- FR 9702118

**Littérature non-brevet citée dans la description**

- **GERAGHTY et al.** *Proc. Natl. Acad. Sci. USA,* 1987, vol. 84, 9145-9149
- **ELLIS et al.** *J. Immunol.,* 1990, vol. 144, 731-735
- **KIRSZENBAUM M. et al.** Oncogeny of hematopoiesis. Aplastic anemia. Colloque INSERM/John Libbey Eurotext Ltd
- **KOVATS et al.** *Science,* 1990, vol. 248, 220-223
- **ISHITANI et al.** *Proc. Natl. Acad. Sci. USA,* 1992, vol. 89, 3947-3951
- **KIRSZENBAUM M. et al.** *Proc. Natl. Acad. Sci. USA,* 1994, vol. 91, 4209-4213
- **KIRSZENBAUM M. et al.** *Human Immunol.,* 1995, vol. 43, 237-241
- **MOREAU P. et al.** *Human Immunol.,* 1995, vol. 43, 231-236
- **KIRSZENBAUM M. et al.** *Human Immunol.,* 1995
- **MOREAU P. et al.** *Human Immunol.,* 1995
- **CAROSELLA E.D. et al.** *C.R. Acad. Sci.,* vol. 318, 827-830
- **CAROSELLA E.D. et al.** *Immunol. Today,* 1996, 407-409
- **CHUMBLEY G. et al.** *Cellular Immunology,* 1994, vol. 155, 312-322
- **AMIOT L. et al.** *Tissue Antigens,* 1996, vol. 48, 609-614
- **CHUMBLEY G. et al.** *Cell Immunol.,* 1994, vol. 155, 312-322
- **DENIZ G. et al.** *J. Immunol.,* 1994, vol. 152, 4255-4261
- **PAZMANY L. et al.** *Science,* 1996, vol. 274, 792-795
- **TEYSSIER M. et al.** *Nat. Immunol.,* 1995, vol. 14, 262-270
- **Colonna M. et al.** *Science,* 1995, vol. 268, 405-408
- **ELLIS et al.** *J. Immunol,* 1990, vol. 144, 731-735
- **KIRSZENBAUM M. et al.** *Hum. Immunol.,* 1995, vol. 43, 237-241
- **MOREAU P. et al.** *Hum. Immunol.,* 1997, vol. 52, 41-46
- **COLONNA et al.** *Science,* 1995, vol. 268, 405-408
- **MORETTA et al.** *J. Exp. Med,* 1994, vol. 180, 545-555
- **CHUMBLEY et al.** *Cell Immunol.,* 1994, vol. 155, 312-322
- **DENIZ et al.** *J. Immunol.,* 1994, vol. 152, 4255-4261
- **COLONNA et al.** *Proc. Natl. Acad. Sci.,* 1992, vol. 89, 7983-7985
- **MANDELBOIM et al.** *J. Exp. Med.,* 1996, vol. 184, 913-922